# EUROPEAN PATENT APPLICATION

(11) **EP 2 001 064 A1**
(43) Date of publication of application: **10.12.2008**
(21) Application number: 07738923.7
(22) Date of filing: 19.03.2007
(51) Int. Cl.: H01L 51/50, C09K 11/06, C07D 307/91, C07D 333/76, C07D 409/14, C07F 15/00

(54) **ORGANIC ELECTROLUMINESCENCE ELEMENT MATERIAL AND ORGANIC ELECTROLUMINESCENCE ELEMENT USING SUCH MATERIAL**

(30) Priority: 24.03.2006 JP 2006082792; 30.03.2006 US 392604
(71) Applicant: Idemitsu Kosan Co., Ltd., Chiyoda-ku Tokyo 100-8321 (JP)
(72) Inventor: NAKANO, Yuki, Sodegaura-shi, Chiba 299-0293 (JP); IWAKUMA, Toshihiro, Sodegaura-shi, Chiba 299-0293 (JP); MATSUURA, Masahide, Sodegaura-shi, Chiba 299-0293 (JP); IKEDA, Hidetsugu, Sodegaura-shi, Chiba 299-0293 (JP)
(74) Representative: Gille Hrabal Struck Neidlein Prop Roos
(86) International application number: PCT/JP2007/055478
(87) International publication number: WO 2007/111176

(57) **Abstract**

A material for organic electroluminescence device with specific structure. An an organic electroluminescence device comprising a cathode, an anode and an organic thin film layer which is sandwiched between the cathode and the anode and comprises at least one layer, wherein at least one layer in the organic thin film layer contains a material for the organic electroluminescence device described above. An organic electroluminescence device with excellent efficiency of light emission, without pixel defects, which is superior in heat resistance and prolonged lifetime is obtained.

## Description

### TECHNICAL FIELD

The present invention relates to a material for an organic electroluminescence device and an organic electroluminescence device employing the same. Particularly, the present invention relates to the material for the organic electroluminescence device with an enhanced efficiency of light emission, free from defects in pixels, superior in heat resistance and with prolonged lifetime, together with the organic electroluminescence device employing the material.

### BACKGROUND ART

An organic electroluminescence ("electroluminescence" will be occasionally referred to as "EL", hereinafter) device is a spontaneous light emitting device which utilizes the principle that a fluorescent substance emits light by energy of recombination of holes injected from an anode and electrons injected from a cathode when an electric field is applied. Since an organic EL device of the laminate type driven under a low electric voltage was reported by C. W. Tang et al. of Eastman Kodak Company (C. W. Tang and S. A. Vanslyke, Applied Physics Letters, Volume 51, Pages 913, 1987), many studies have been conducted on organic EL devices using organic materials as the constituting materials. Tang et al. used a laminate structure using tris(8-hydroxyquinolinol aluminum) for the light emitting layer and a triphenyldiamine derivative for the hole transporting layer. Advantages of the laminate structure are that the efficiency of hole injection into the light emitting layer can be increased, that the efficiency of forming excited particles which are formed by blocking and recombining electrons injected from the cathode can be increased, and that excited particles formed among the light emitting layer can be enclosed. As the structure of the organic EL device, a two-layered structure having a hole transporting (injecting) layer and an electron transporting and light emitting layer and a three-layered structure having a hole transporting (injecting) layer, a light emitting layer and an electron transporting (injecting) layer are well known. To increase the efficiency of recombination of injected holes and electrons in the devices of the laminate type, the structure of the device and the process for forming the device have been studied.

As the light emitting material of the organic EL device, chelate complexes such as tris(8-quinolinolato)aluminum, coumarine derivatives, tetraphenylbutadiene derivatives, bisstyrylarylene derivatives and oxadiazole derivatives are known. It is reported that light in the visible region ranging from blue light to red light can be obtained by using these light emitting materials, and development of a device exhibiting color images is expected (refer to, for example, Patent Literatures 1 to 3 below).
Further, in late years, employing of a phosphorescent material other than the fluorescent material as the light emitting layer of the organic EL device is proposed (refer to, for example, Non-patent Literatures 1 and 2 below). As described above, a great efficiency of light emission is achieved by utilizing an organic phosphorescent material excited to the singlet state and the triplet state in the light emitting layer of an organic EL device. It is considered that singlet excimers and triplet excimers are formed in relative amounts of 1:3 due to the difference in the multiplicity of spin when electrons and holes are recombined in an organic EL device. Therefore, it is expected that an efficiency of light emission 3 to 4 times as great as that of a device utilizing fluorescence alone can be achieved by utilizing a phosphorescent light emitting material.

In the organic EL devices such as those described above, constructions in which layers such as an anode, an organic light emitting layer, an electron transporting layer (a hole blocking layer), an electron injecting layer and a cathode are successively laminated are used so that light emission in the condition excited to the triplet state or from excimers in the triplet state is not quenched. In the organic light emitting layer, a host compound and the phosphorescent light emitting compound are employed (refer to, for example, Patent Literature 4 below). Namely, Patent Literature 4 discloses host materials having dibenzofuran skeleton or dibenzothiophene skeleton. However, Patent Literature 4 fails to clarify a superiority in device performance over other carbazolyl skeletons and further, it does not describe about a substituent at 2, 8-positions of dibenzofuran or of dibenzothiophene. Also, Patent Literature 4 is silent about that the substituent at 2, 8-positions do not degrade a value of a triplet energy gap and that they have a remarkable superiority as a host material or other transport material for a phosphorus optical device.

Further, an organic EL device comprising a fluorescent benzofuran compound or a fluorescent dibenzofuran compound is disclosed (refer to Patent Literature 5 below). However, Patent Literature 5 neither describes about substituent at 7-position of benzofuran and at 2 or 8-position of dibenzofuran nor describes about superiority of the substituents.

Furthermore, a compound having benzothiophene skeleton of which anthracene skeleton is essential is described (refer to Patent Literature 6 below). However, the compound seems to be difficult in applying to a phosphorus luminescent device because it has the anthracene skeleton with a narrow triplet energy gap. Moreover, although Patent Literature 7 below indicates benzofuran compound bonded to pyrene skeleton, the compound also seems to be difficult in applying to the phosphorus luminescent device because the pyrene skeleton has also a narrow triplet energy gap, and further, Patent Literature 7 fails to describe any example about the compound.
Still further, a compound whose 2-position of dibenzothiophene and of dibenzofuran are substituted with phenyl group is described (refer to Patent Literature 8 below). However, Patent Literature 8 is silent about either an example of a phosphorus luminescent device employing the compound or about that the substituents at 2 and 8-positions do not deteriorate the value of the triplet energy gap and that the compound is superior as a host material or as another transporting material for the phosphorus luminescent device, particularly as a material for a luminescent device having a light emitting wavelength of the EL device shorter than 520 nm.

Still further, there are descriptions about a compound having a substituent such as arylsilyl group or so (refer to Patent Literatures 9 and 10). However, there is not any description about compounds relating to the present invention in Patent Literatures 9 and 10, further to say nothing about a profitable effect as a material for an EL device, particularly as a material for a bluish phosphorus luminescent device such that the compound keeps the triplet energy gap broad.
Still further, there are descriptions about arylsilane-based compound or about arylgermane-based compound (refer to Patent Literatures 11 to 17). Although there are examples about host materials for a bluish phosphorus luminescent device disclosed in some of Patent Literatures 11 to 17, they are silent either about the compounds relating to the present invention or about their effects.

Patent Literature 1: Japanese Unexamined Patent Application Laid-Open No. Heisei 8(1996)-239655
Patent Literature 2: Japanese Unexamined Patent Application Laid-Open No. Heisei 7(1995)-138561
Patent Literature 3: Japanese Unexamined Patent Application Laid-Open No. Heisei 3(1991)-200889
Patent Literature 4: International PCT Publication No. WO 05/101912
Patent Literature 5: Japanese Unexamined Patent Application Laid-Open No. Heisei 5(1993)-109485
Patent Literature 6: Japanese Unexamined Patent Application Laid-Open No. 2004-002351
Patent Literature 7: International PCT Publication No. WO 04/096945
Patent Literature 8: Japanese Unexamined Patent Application Laid-Open No. 2002-308837
Patent Literature 9: Japanese Unexamined Patent Application Laid-Open No. 2003-138251
Patent Literature 10: Japanese Unexamined Patent Application Laid-Open No. 2000-351966
Patent Literature 11: International PCT Publication No. WO 2004/095598
Patent Literature 12: U. S. Patent Publication No. 2004/209115 A1
Patent Literature 13: Japanese Unexamined Patent Application Laid-Open No. 2004-103463
Patent Literature 14: Japanese Unexamined Patent Application Laid-Open No. 2005-183303
Patent Literature 15: Japanese Unexamined Patent Application Laid-Open No. 2005-317275
Patent Literature 16: Japanese Unexamined Patent Application Laid-Open No. 2004-200104
Patent Literature 17: Japanese Unexamined Patent Application Laid-Open No. 2003-243178
Non-patent Literature 1: D.F.O'Brien and M.A.Baldo et al. "Improved energy transfering electrophosphorescent devices" Applied Physics letters Vol. 74 No. 3, pp442-444, January 18, 1999
Non-patent Literature 2: M.A.Baldo et al. "Very high-efficiency green organic light-emitting devices based on electrophosphorescence" Applied Physics letters Vol. 75 No. 1, pp4-6, July 5, 1999

### DISCLOSURE OF THE INVENTION

The present invention has been made to overcome the above problems and has an object of providing a material for the organic electroluminescence device with an enhanced efficiency of light emission, free from defects in pixels, superior in heat resistance and with prolonged lifetime, together with the organic electroluminescence device employing the material.

As a result of intensive and extensive researches for overcoming the above problems, the inventors have found that using a compound represented by a general formula (1) below as a material enables to achieve an organic EL device with an enhanced efficiency of light emission, free from defects in pixels, superior in heat resistance and with prolonged lifetime. The present invention has been accomplished on the basis of the above finding.

Namely, the present invention provides a material for an organic electroluminescence device comprising a compound represented by a following general formula (1): 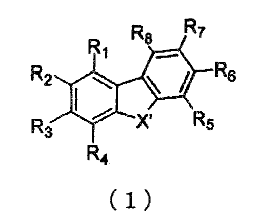 wherein R₁ to R₈ each independently represents a hydrogen atom, a halogen atom, an alkyl group having 1 to 40 carbon atoms and further may have a substituent, a heterocyclic group except pyridine ring while the heterocyclic group has 3 to 20 carbon atoms and further may have a substituent, an alkoxy group having 1 to 40 carbon atoms and further may have a substituent, a non-condensed aryl group having 6 to 40 carbon atoms and further may have a substituent, a condensed aryl group having 6 to 12 carbon atoms and further may have a substituent, a mixed aryl group of a condensed aryl group and a non-condensed aryl group while the mixed aryl group has 12 to 40 carbon atoms and further may have a substituent, an aryloxy group having 6 to 20 carbon atoms and further may have a substituent, an aralkyl group having 7 to 20 carbon atoms and further may have a substituent, an alkenyl group having 2 to 40 carbon atoms and further may have a substituent, an alkylamino group having 1 to 40 carbon atoms and further may have a substituent, an aralkylamino group having 7 to 60 carbon atoms and further may have a substituent, an alkylsilyl group having 3 to 20 carbon atoms and further may have a substituent, an arylsilyl group having 8 to 40 carbon atoms and further may have a substituent, an aralkylsilyl group having 8 to 40 carbon atoms and further may have a substituent, an alkylgermanium group having 3 to 20 carbon atoms and further may have a substituent, an arylgermanium group having 8 to 40 carbon atoms and further may have a substituent, an aralkylgermanium group having 8 to 40 carbon atoms and further may have a substituent, a keto aryl group having 7 to 40 carbon atoms and further may have a substituent, an alkylhalide group having 1 to 40 carbon atoms and further may have a substituent, or a cyano group;
X' represents a sulfur atom, an oxygen atom or a substituted germanium group expressed by GₑR_{c}R_{d}, while R_{c} and R_{d} each independently represents an alkyl group having 1 to 40 carbon atoms or an aryl group having 6 to 20 carbon atoms; however, at least one of R₂ or R₇ independently represents a non-fused aromatic ring having 6 to 40 carbon atoms and further may have a substituent, a naphthyl group which may have a substituent, an alkylsilyl group having 3 to 20 carbon atoms and further may have a substituent, an arylsilyl group having 8 to 40 carbon atoms and further may have a substituent, an aralkylsilyl group having 8 to 40 carbon atoms and further may have a substituent, an alkylgermanium group having 3 to 20 carbon atoms and further may have a substituent, an arylgermanium group having 8 to 40 carbon atoms and further may have a substituent or an aralkylgermanium group having 8 to 40 carbon atoms and further may have a substituent; and
each of R₂ and R₇ is not an amino group.

Further, the present invention provides a material for an organic electroluminescence device comprising a compound represented by a following general formula (2) or a following general formula (3): 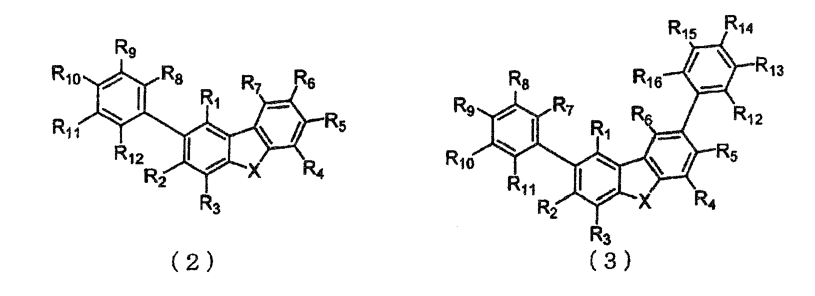 wherein R₁ to R₁₆ each independently represents a hydrogen atom, a halogen atom, an alkyl group having 1 to 40 carbon atoms and further may have a substituent, a heterocyclic group having 3 to 20 carbon atoms and further may have a substituent, an alkoxy group having 1 to 40 carbon atoms and further may have a substituent, a non-condensed aryl group having 6 to 40 carbon atoms and further may have a substituent, a condensed aryl group having 6 to 12 carbon atoms and further may have a substituent, a mixed aryl group of a condensed aryl group and a non-condensed aryl group while the mixed aryl group has 12 to 40 carbon atoms and further may have a substituent, an aryloxy group having 6 to 20 carbon atoms and further may have a substituent, an aralkyl group having 7 to 20 carbon atoms and further may have a substituent, an alkenyl group having 2 to 40 carbon atoms and further may have a substituent, an alkylamino group having 1 to 40 carbon atoms and further may have a substituent, an aralkylamino group having 7 to 60 carbon atoms and further may have a substituent, an alkylsilyl group having 3 to 20 carbon atoms and further may have a substituent, an arylsilyl group having 8 to 40 carbon atoms and further may have a substituent, an aralkylsilyl group having 8 to 40 carbon atoms and further may have a substituent, an alkylgermanium group having 3 to 20 carbon atoms and further may have a substituent, an arylgermanium group having 8 to 40 carbon atoms and further may have a substituent, an aralkylgermanium group having 8 to 40 carbon atoms and further may have a substituent, a keto aryl group having 7 to 40 carbon atoms and further may have a substituent, an alkylhalide group having 1 to 40 carbon atoms and further may have a substituent, or a cyano group;
X represents a sulfur atom, an oxygen atom, a substituted silicon atom expressed by SiRₐR_{b} or a substituted germanium group expressed by GeR_{c}R_{d}, while Rₐ, R_{b}, R_{c} and R_{d} each independently represents an alkyl group having 1 to 40 carbon atoms or an aryl group having 6 to 20 carbon atoms;
however, each of R₁₀ in the general formula (2), R₉ and R₁₄ in the general formula (3) is not an amino group; and
at least one of R₈ or R₁₂ in the general formula (2) is a hydrogen atom.

Furthermore, the present invention provides a material for an organic electroluminescence device comprising a compound represented by any one of following general formula (6) or general formula (7): 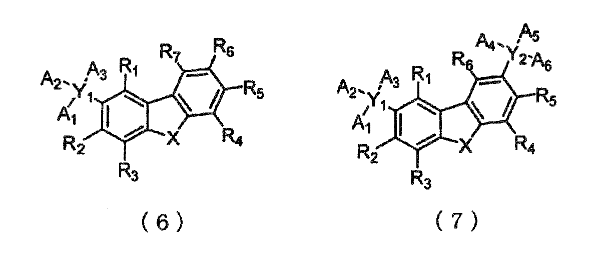 wherein R₁ to R₇ each independently represents a hydrogen atom, a halogen atom, an alkyl group having 1 to 40 carbon atoms and further may have a substituent, a heterocyclic group having 3 to 20 carbon atoms and further may have a substituent, an alkoxy group having 1 to 40 carbon atoms and further may have a substituent, a non-condensed aryl group having 6 to 40 carbon atoms and further may have a substituent, a condensed aryl group having 6 to 12 carbon atoms and further may have a substituent, a mixed aryl group of a condensed aryl group and a non-condensed aryl group while the mixed aryl group has 12 to 40 carbon atoms and further may have a substituent, an aryloxy group having 6 to 20 carbon atoms and further may have a substituent, an aralkyl group having 7 to 20 carbon atoms and further may have a substituent, an alkenyl group having 2 to 40 carbon atoms and further may have a substituent, an alkylamino group having 1 to 40 carbon atoms and further may have a substituent, an aralkylamino group having 7 to 60 carbon atoms and further may have a substituent, an alkylsilyl group having 3 to 20 carbon atoms and further may have a substituent, an arylsilyl group having 8 to 40 carbon atoms and further may have a substituent, an aralkylsilyl group having 8 to 40 carbon atoms and further may have a substituent, an alkylgermanium group having 3 to 20 carbon atoms and further may have a substituent, an arylgermanium group having 8 to 40 carbon atoms and further may have a substituent, an aralkylgermanium group having 8 to 40 carbon atoms and further may have a substituent, a keto aryl group having 7 to 40 carbon atoms and further may have a substituent, an alkylhalide group having 1 to 40 carbon atoms and further may have a substituent, or a cyano group;
X represents a sulfur atom, an oxygen atom, a substituted silicon atom expressed by SiRₐR_{b} or a substituted germanium group expressed by GₑR_{c}R_{d}, while Rₐ, R_{b}, R_{c} and R_{d} each independently represents an alkyl group having 1 to 40 carbon atoms or an aryl group having 6 to 20 carbon atoms;
Y₁ and Y₂ each independently represents a silicon atom or a germanium atom; and A₁ to A₆ each independently represents an alkyl group having 1 to 40 carbon atoms and further may have a substituent, an aryl group having 6 to 40 carbon atoms and further may have a substituent or an aralkyl group having 7 to 20 carbon atoms and further may have a substituent.
Further, the present invention provides an organic electroluminescence device comprising an anode, a cathode and at least one organic thin film layer having a light emitting layer sandwiched between the anode and the cathode, wherein at least one of the organic thin film layer comprises the foregoing material for an organic electroluminescence device.

### EFFECTS OF THE INVENTION

Employing the compound represented by the general formula (1) of the present invention as the material for an organic electroluminescence device provides the organic electroluminescence device with an enhanced current efficiency of light emission, without any pixel defects, with superiority in heat resistance and with prolonged lifetime.
Therefore, the organic EL device of the present invention is very useful for applications such as light sources of various electronic instruments.

### THE PREFERRED EMBODIMENT TO CARRY OUT THE INVENTION

The present invention provides a material for an organic electroluminescence device comprising a compound represented by a following general formula (1): 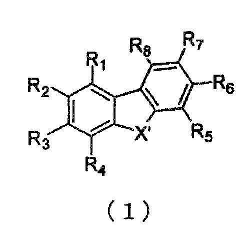 wherein R₁ to R₈ each independently represents a hydrogen atom, a halogen atom, an alkyl group having 1 to 40 carbon atoms and further may have a substituent, a heterocyclic group except pyridine ring while the heterocyclic group has 3 to 20 carbon atoms and further may have a substituent, an alkoxy group having 1 to 40 carbon atoms and further may have a substituent, a non-condensed aryl group having 6 to 40 carbon atoms and further may have a substituent, a condensed aryl group having 6 to 12 carbon atoms and further may have a substituent, a mixed aryl group of a condensed aryl group and a non-condensed aryl group while the mixed aryl group has 12 to 40 carbon atoms and further may have a substituent, an aryloxy group having 6 to 20 carbon atoms and further may have a substituent, an aralkyl group having 7 to 20 carbon atoms and further may have a substituent, an alkenyl group having 2 to 40 carbon atoms and further may have a substituent, an alkylamino group having 1 to 40 carbon atoms and further may have a substituent, an aralkylamino group having 7 to 60 carbon atoms and further may have a substituent, an alkylsilyl group having 3 to 20 carbon atoms and further may have a substituent, an arylsilyl group having 8 to 40 carbon atoms and further may have a substituent, an aralkylsilyl group having 8 to 40 carbon atoms and further may have a substituent, an alkylgermanium group having 3 to 20 carbon atoms and further may have a substituent, an arylgermanium group having 8 to 40 carbon atoms and further may have a substituent, an aralkylgermanium group having 8 to 40 carbon atoms and further may have a substituent, a keto aryl group having 7 to 40 carbon atoms and further may have a substituent, an alkylhalide group having 1 to 40 carbon atoms and further may have a substituent, or a cyano group;
X' represents a sulfur atom, an oxygen atom or a substituted germanium group expressed by GeR_{c}R_{d}, while R_{c} and R_{d} each independently represents an alkyl group having 1 to 40 carbon atoms or an aryl group having 6 to 20 carbon atoms; however, at least one of R₂ or R₇ independently represents a non-fused aromatic ring having 6 to 40 carbon atoms and further may have a substituent, a naphthyl group which may have a substituent, an alkylsilyl group having 3 to 20 carbon atoms and further may have a substituent, an arylsilyl group having 8 to 40 carbon atoms and further may have a substituent, an aralkylsilyl group having 8 to 40 carbon atoms and further may have a substituent, an alkylgermanium group having 3 to 20 carbon atoms and further may have a substituent, an arylgermanium group having 8 to 40 carbon atoms and further may have a substituent or an aralkylgermanium group having 8 to 40 carbon atoms and further may have a substituent; and
each of R₂ and R₇ is not an amino group.

The present invention provides a material for an organic electroluminescence device which comprises a compound represented by a following general formula (2) or a following general formula (3): 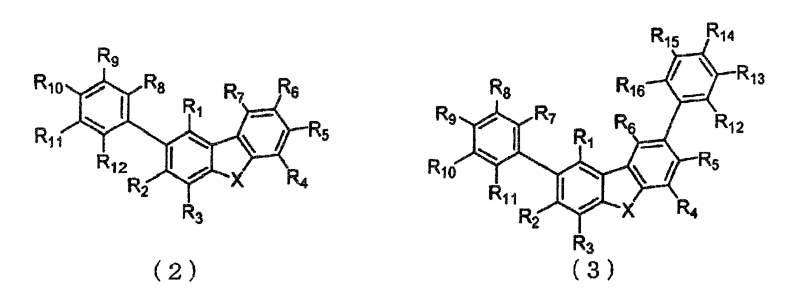 R₁ to R₁₆ in the general formulae (2) and (3) each independently represents a hydrogen atom, a halogen atom, an alkyl group having 1 to 40 carbon atoms and further may have a substituent, a heterocyclic group having 3 to 20 carbon atoms and further may have a substituent, an alkoxy group having 1 to 40 carbon atoms and further may have a substituent, a non-condensed aryl group having 6 to 40 carbon atoms and further may have a substituent, a condensed aryl group having 6 to 12 carbon atoms and further may have a substituent, a mixed aryl group of a condensed aryl group and a non-condensed aryl group while the mixed aryl group has 12 to 40 carbon atoms and further may have a substituent, an aryloxy group having 6 to 20 carbon atoms and further may have a substituent, an aralkyl group having 7 to 20 carbon atoms and further may have a substituent, an alkenyl group having 2 to 40 carbon atoms and further may have a substituent, an alkylamino group having 1 to 40 carbon atoms and further may have a substituent, an aralkylamino group having 7 to 60 carbon atoms and further may have a substituent, an alkylsilyl group having 3 to 20 carbon atoms and further may have a substituent, an arylsilyl group having 8 to 40 carbon atoms and further may have a substituent, an aralkylsilyl group having 8 to 40 carbon atoms and further may have a substituent, an alkylgermanium group having 3 to 20 carbon atoms and further may have a substituent, an arylgermanium group having 8 to 40 carbon atoms and further may have a substituent, an aralkylgermanium group having 8 to 40 carbon atoms and further may have a substituent, a keto aryl group having 7 to 40 carbon atoms and further may have a substituent, an alkylhalide group having 1 to 40 carbon atoms and further may have a substituent, or a cyano group.
X in the general formulae (2) and (3) each independently represents a sulfur atom, an oxygen atom, a substituted silicon atom expressed by SiRₐR_{b} or a substituted germanium group expressed by GeR_{c}R_{d}, while Rₐ, R_{b}, R_{c} and R_{d} each independently represents an alkyl group having 1 to 40 carbon atoms or an aryl group having 6 to 20 carbon atoms;
however, each of R₁₀ in the general formula (2), R₉ and R₁₄ in the general formula (3) is not an amino group; and
at least one of R₈ or R₁₂ in the general formula (2) is a hydrogen atom.

It is preferable that the material for the organic EL device represented by the above general formula (2) is a compound expressed by a following general formula (4) or a following general formula (5). 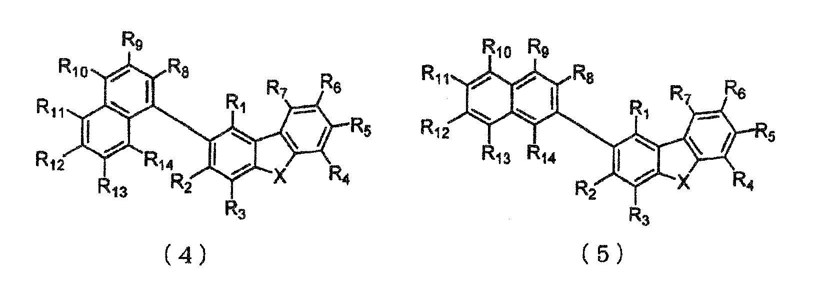 R₁ to R₁₄ in the general formulae (4) and (5) each independently represents the same as R₁ to R₁₆ in the general formulae (2) and (3). Further, X in the general formulae (4) and (5) is also the same as X in the general formula (2).

The present invention provides a material for an organic electroluminescence device which comprises a compound represented by a following general formula (6) or a following general formula (7): 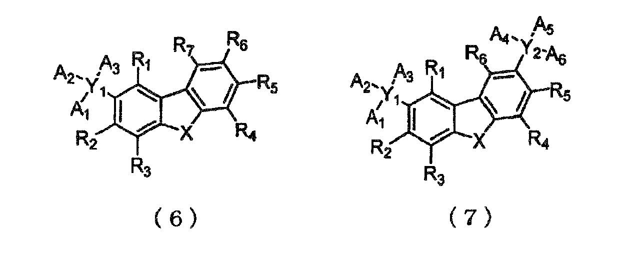 R₁ to R₇ in the general formulae (6) and (7) each independently represents the same as R₁ to R₁₆ in the general formulae (2) and (3). Further, X in the general formulae (6) and (7) is also the same as X in the general formulae (2) and (3).
Y₁ and Y₂ in the general formulae (6) and (7) each independently represents a silicon atom or a germanium atom; and
A₁ to A₆ each independently represents an alkyl group having 1 to 40 carbon atoms and further may have a substituent, an aryl group having 6 to 40 carbon atoms and further may have a substituent or an aralkyl group having 7 to 20 carbon atoms and further may have a substituent.
The material for an organic electroluminescence device preferably comprises a compound represented by the general formula (1) wherein at least one selected from R₁ to R₈ is a mono-valent group derived from dibenzofuran, dibenzothiophene, carbazole, silafluorene, germafluorene or fluorene.
The material for an organic electroluminescence device preferably comprises a compound represented by the general formula (1) wherein at least one of R₁ and R₇ is a mono-valent group derived from dibenzofuran, dibenzothiophene, carbazole, silafluorene, germafluorene or fluorene.

Examples of the halogen atom represented by R₁ to R₁₆ in the general formulae (1) to (7) include fluorine atom, chlorine atom, bromine atom, iodine atom, etc.
Examples of the alkyl group represented by R₁ to R₁₆ each having 1 to 40 carbon atoms and further may have a substituent include methyl group, ethyl group, propyl group, isopropyl group, n-butyl group, s-butyl group, isobutyl group, t-butyl group, n-pentyl group, n-hexyl group, n-heptyl group, n-octyl group, n-nonyl group, n-decyl group, n-undecyl group, n-dodecyl group, n-tridecyl group, n-tetradecyl group, n-pentadecyl group, n-hexadecyl group, n-heptadecyl group, n-octadecyl group, neopentyl group, 1-methylpentyl group, 2-methylpentyl group, 1-pentylhexyl group, 1-butylpentyl group, 1-heptyloctyl group, 3-methylpentyl group, hydroxymethyl group, 1-hydroxyethyl group, 2-hydroxyethyl group, 2-hydroxyisobutyl group, 1,2-dihydroxyethyl group, 1,3-dihydroxy isopropyl group, 2,3-dihydroxy-t-butyl group, 1,2,3-trihydroxypropyl group, chloromethyl group, 1-chloroethyl group, 2-chloroethyl group, 2-chloro isobutyl group, 1,2-dichloroethyl group, 1,3-dichloro isopropyl group, 2,3-dichloro-t-butyl group, 1,2,3-trichloro propyl group, bromomethyl group, 1-bromoethyl group, 2-bromoethyl group, 2-bromo isobutyl group, 1,2-dibromo ethyl group, 1,3-dibromo isopropyl group, 2,3-dibromo-t-butyl group, 1,2,3-tribromo propyl group, iodo methyl group, 1-iodo ethyl group, 2-iodo ethyl group, 2-iodo isobutyl group, 1,2-diiodo ethyl group, 1,3-diiodo isopropyl group, 2,3-diiodo-t-butyl group, 1,2,3-triiodo propyl group, aminomethyl group, 1-amino ethyl group, 2-amino ethyl group, 2-amino isobutyl group, 1,2-diamino ethyl group, 1,3-diamino isopropyl group, 2,3-diamino-t-butyl group, 1,2,3-triamino propyl group, cyanomethyl group, 1-cyanoethyl group, 2-cyanoethyl group, 2-cyano isobutyl group, 1,2-dicyano ethyl group, 1,3-dicyano isopropyl group, 2,3-dicyano-t-butyl group, 1,2,3-tricyanopropyl group, nitromethyl group, 1-nitroethyl group, 2-nitroethyl group, 1,2-dinitroethyl group, 2,3-dinitro-t-butyl group, 1,2,3-trinitro propyl group, cyclopentyl group, cyclohexyl group, cyclo octyl group, 3,5-tetramethyl cyclohexyl group, etc.

Among those, methyl group, ethyl group, propyl group, isopropyl group, n-butyl group, s-butyl group, isobutyl group, t-butyl group, n-pentyl group, n-hexyl group, n-heptyl group, n-octyl group, n-nonyl group, n-decyl group, n-undecyl group, n-dodecyl group, n-tridecyl group, n-tetradecyl group, n-pentadecyl group, n-hexadecyl group, n-heptadecyl group, n-octadecyl group, neopentyl group, 1-methylpentyl group, 1-pentylhexyl group, 1-butylpentyl group, 1-heptyloctyl group, cyclohexyl group, cyclo octyl group and 3,5-tetramethyl cyclohexyl group are preferable.

Examples of the above heterocyclic group represented by R₁ to R₁₆ each having 3 to 20 carbon atoms and further may have a substituent in the foregoing general formulae (2) to (7) include 1-pyrrolyl group, 2-pyrrolyl group, 3-pyrrolyl group, pyrazinyl group, 2-pyridinyl group, 1-imidazolyl group, 2-imidazolyl group, 1-pyrazolyl group, 1-indolizinyl group, 2-indolizinyl group, 3-indolizinyl group, 5-indolizinyl group, 6-indolizinyl group, 7-indolizinyl group, 8-indolizinyl group, 2-imidazopyridinyl group, 3-imidazopyridinyl group, 5-imidazopyridinyl group, 6-imidazopyridinyl group, 7-imidazopyridinyl group, 8-imidazopyridinyl group, 3-pyridinyl group, 4-pyridinyl group, 1-indolyl group, 2-indolyl group, 3-indolyl group, 4-indolyl group, 5-indolyl group, 6-indolyl group, 7-indolyl group, 1-iso indolyl group, 2-iso indolyl group, 3-iso indolyl group, 4-iso indolyl group, 5-iso indolyl group, 6-iso indolyl group, 7-iso indolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, 2-quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxalinyl group, 5-quinoxalinyl group, 6-quinoxalinyl group, 1-carbazolyl group, 2-carbazolyl group, 3-carbazolyl group, 4-carbazolyl group, 9-carbazolyl group, β-carboline-1-yl group, β-carboline-3-yl group, β-carboline-4-yl group, β-carboline-5-yl group, β-carboline-6-yl group, β -carboline-7-yl group, β -carboline-8-yl group, β -carboline-9-yl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthroline-2-yl group, 1,7-phenanthroline-3-yl group, 1,7-phenanthroline-4-yl group, 1,7-phenanthroline-5-yl group, 1,7-phenanthroline-6-yl group, 1,7-phenanthroline-8-yl group, 1,7-phenanthroline-9-yl group, 1,7-phenanthroline-10-yl group, 1,8-phenanthroline-2-yl group, 1,8-phenanthroline-3-yl group, 1,8-phenanthroline-4-yl group, 1,8-phenanthroline-5-yl group, 1,8-phenanthroline-6-yl group, 1,8-phenanthroline-7-yl group, 1,8-phenanthroline-9-yl group, 1,8-phenanthroline-10-yl group, 1,9-phenanthroline-2-yl group, 1,9-phenanthroline-3-yl group, 1,9-phenanthroline-4-yl group, 1,9-phenanthroline-5-yl group, 1,9-phenanthroline-6-yl group, 1,9-phenanthroline-7-yl group, 1,9-phenanthroline-8-yl group, 1,9-phenanthroline-10-yl group, 1,10-phenanthroline-2-yl group, 1,10-phenanthroline-3-yl group,1,10-phenanthroline-4-yl group, 1,10-phenanthroline-5-yl group,2,9-phenanthroline-1-yl group, 2,9-phenanthroline-3-yl group, 2,9-phenanthroline-4-yl group, 2,9-phenanthroline-5-yl group, 2,9-phenanthroline-6-yl group, 2,9-phenanthroline-7-yl group, 2,9-phenanthroline-8-yl group, 2,9-phenanthroline-10-yl group, 2,8-phenanthroline-1-yl group, 2,8-phenanthroline-3-yl group, 2,8-phenanthroline-4-yl group, 2,8-phenanthroline-5-yl group, 2,8-phenanthroline-6-yl group, 2,8-phenanthroline-7-yl group, 2,8-phenanthroline-9-yl group, 2,8-phenanthroline-10-yl group, 2,7-phenanthroline-1-yl group, 2,7-phenanthroline-3-yl group, 2,7-phenanthroline-4-yl group, 2,7-phenanthroline-5-yl group, 2,7-phenanthroline-6-yl group, 2,7-phenanthroline-8-yl group, 2,7-phenanthroline-9-yl group, 2,7-phenanthroline-10-yl group, 1-phenazinyl group, 2-phenazinyl group, 1-phenothiazinyl group, 2-phenothiazinyl group, 3-phenothiazinyl group, 4-phenothiazinyl group, 10-phenothiazinyl group, 1-phenoxazinyl group, 2-phenoxazinyl group, 3-phenoxazinyl group, 4-phenoxazinyl group, 10-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrole-1-yl group, 2-methylpyrrole-3-yl group, 2-methylpyrrole-4-yl group, 2-methylpyrrole-5-yl group, 3-methylpyrrole-1-yl group, 3-methylpyrrole-2-yl group, 3-methylpyrrole-4-yl group, 3-methylpyrrole-5-yl group, 2-t-butylpyrrole-4-yl group, 3-(2-phenylpropyl) pyrrole-1-yl group, 2-methyl-1-indolyl group, 4-methyl-1-indolyl group, 2-methyl-3-indolyl group, 4-methyl-3-indolyl group, 2-t-butyl 1-indolyl group, 4-t-butyl 1-indolyl group, 2-t-butyl 3-indolyl group, 4-t-butyl 3-indolyl group, 1-dibenzofuranyl group, 2-dibenzofuranyl group, 3-dibenzofuranyl group, 4-dibenzofuranyl group, 1-dibenzothiophenyl group, 2-dibenzothiophenyl group, 3-dibenzothiophenyl group, 4-dibenzothiophenyl group, 1-silafluorenyl group, 2-silafluorenyl group, 3-silafluorenyl group, 4-silafluorenyl group, 1-germafluorenyl group, 2-germafluorenyl group, 3-germafluorenyl group, 4-germafluorenyl group, etc.

Among those, 2-pyridinyl group, 1-indolizinyl group, 2-indolizinyl group, 3-indolizinyl group, 5-indolizinyl group, 6-indolizinyl group, 7-indolizinyl group, 8-indolizinyl group, 2-imidazopyridinyl group, 3-imidazopyridinyl group, 5-imidazopyridinyl group, 6-imidazopyridinyl group, 7-imidazopyridinyl group, 8-imidazopyridinyl group, 3-pyridinyl group, 4-pyridinyl group, 1-indolyl group, 2-indolyl group, 3-indolyl group, 4-indolyl group, 5-indolyl group, 6-indolyl group, 7-indolyl group, 1-iso indolyl group, 2-iso indolyl group, 3-iso indolyl group, 4-iso indolyl group, 5-iso indolyl group, 6-iso indolyl group, 7-iso indolyl group, 1-carbazolyl group, 2-carbazolyl group, 3-carbazolyl group, 4-carbazolyl group 9-carbazolyl group, 1-dibenzofuranyl group, 2-dibenzofuranyl group, 3-dibenzofuranyl group, 4-dibenzofuranyl group, 1-dibenzothiophenyl group, 2-dibenzothiophenyl group, 3-dibenzothiophenyl group, 4-dibenzothiophenyl group, 1-silafluorenyl group, 2-silafluorenyl group, 3-silafluorenyl group, 4-silafluorenyl group, 1-germafluorenyl group, 2-germafluorenyl group, 3-germafluorenyl group and 4-germafluorenyl group are preferable.
Examples of the heterocyclic group except pyridine ring while the heterocyclic group has 3 to 20 carbon atoms and further may have a substituent that are represented by R₁ to R₈ in the general formula (1) are the above examples of the heterocyclic group excluding 2-pyridinyl group, 3-pyridinyl group and 4-pyridinyl group, including the preferable examples.

The alkoxy group represented by R₁ to R₁₆ each having 1 to 40 carbon atoms and further may have a substituent in the general formulae (1) to (7) is expressed by -OY and examples of Y are the same as described about the foregoing alkyl group including the preferable examples.

Examples of the non-condensed aryl group having 6 to 40 carbon atoms and further may have a substituent that are represented by R₁ to R₁₆ in the general formulae (1) to (7) include phenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 4'-methylbiphenylyl group, 4"-t-butyl-p-terphenyl-4-yl group, o-cumenyl group, m-cumenyl group, p-cumenyl group, 2,3-xylyl group, 3,4-xylyl group, 2,5-xylyl group, mesityl group, m-quarterphenyl group, etc.
Among those, preferable examples are phenyl group, 2-biphenylyl group, 3-biphenylyl group, 4-biphenylyl group, m-terphenyl-4-yl group, m-terphenyl-3-yl group, m-terphenyl-2-yl group, p-tolyl group, 3,4-xylyl group, m-quarterphenyl-2-yl group, etc.
Examples of the condensed aryl group having 6 to 12 carbon atoms and further may have a substituent that are represented by R₁ to R₁₆ in the general formulae (1) to (7) include 1-naphthyl group and 2-naphthyl group.
Examples of the mixed aryl group of a condensed aryl group and a non-condensed aryl group while the mixed aryl group has 12 to 40 carbon atoms and further may have a substituent that are represented by R₁ to R₁₆ in the general formulae (1) to (7) include a group made by combining the above examples of the non-condensed aryl group having 6 to 40 carbon atoms and further may have a substituent and the above examples of the condensed aryl group having 6 to 12 carbon atoms and further may have a substituent.

The aryloxy group having 6 to 20 carbon atoms and further may have a substituent that are represented by R₁ to R₁₆ in the general formulae (1) to (7) is expressed by -OAr and specific examples of Ar are the same as the above examples explained about the above non-condensed aryl group including the preferable examples.

Examples of the aralkyl group having 7 to 20 carbon atoms and further may have a substituent that are represented by R₁ to R₁₆ in the general formulae (1) to (7) include benzyl group, 1-phenylethyl group, 2-phenylethyl group, 1-phenylisopropyl group, 2-phenylisopropyl group, phenyl-t-butyl group, α -naphthylmethyl group, 1-α-naphthylethyl group, 2-α-naphthylethyl group, 1-α-naphthylisopropyl group, 2-α-naphthylisopropyl group, β-naphthylmethyl group, 1- β -naphthylethyl group, 2- β -naphthylethyl group, 1- β -naphthylisopropyl group, 2-β-naphthylisopropyl group, 1-pyrrolylmethyl group, 2-(1-pyrrolyl)ethyl group, p-methylbenzyl group, m-methylbenzyl group, o-methylbenzyl group, p-chlorobenzyl group, m-chlorobenzyl group, o-chlorobenzyl group, p-bromobenzyl group, m-bromobenzyl group, o-bromobenzyl group, p-iodobenzyl group, m-iodobenzyl group, o-iodobenzyl group, p-hydroxybenzyl group, m-hydroxybenzyl group, o-hydroxybenzyl group, p-aminobenzyl group, m-aminobenzyl group, o-aminobenzyl group, p-nitrobenzyl group, m-nitrobenzyl group, o-nitrobenzyl group, p-cyanobenzyl group, m-cyanobenzyl group, o-cyano benzyl group, 1-hydroxy-2-phenylisopropyl group, 1-chloro-2-phenylisopropyl group, etc.
Among those, benzyl group, p-cyanobenzyl group, m-cyano benzyl group, o-cyanobenzyl group, 1-phenylethyl group, 2-phenylethyl group, 1-phenylisopropyl group and 2-phenylisopropyl group are preferable.

Examples of the alkenyl group having 2 to 40 carbon atoms and further may have a substituent that are represented by R₁ to R₁₆ in the general formulae (1) to (7) include vinyl group, aryl group, 1-butenyl group, 2-butenyl group, 3-butenyl group, 1,3-butanedienyl group, 1-methylvinyl group, styryl group, 2,2-diphenylvinyl group, 1,2-diphenylvinyl group, 1-methylaryl group, 1,1-dimethylaryl group, 2-methylaryl group, 1-phenylaryl group, 2-phenylaryl group, 3-phenylaryl group, 3,3-diphenylaryl group, 1,2-dimethylaryl group, 1-phenyl-1-butenyl group, 3-phenyl-1-butenyl group and so on, while styryl group, 2,2-diphenylvinyl group and 1,2-diphenylvinyl group are preferable.

The alkylamino group having 1 to 40 carbon atoms and further may have a substituent and the aralkylamino group having 7 to 60 carbon atoms and further may have a substituent that are represented by R₁ to R₁₆ in the general formulae (1) to (7) are expressed by -NQ₁Q₂, and specific examples of Q₁ and Q₂ each independently represents the same examples as explained about the above alkyl group, the above aryl group and the above aralkyl group including the preferable examples.
Examples of the alkylsilyl group having 3 to 20 carbon atoms and further may have a substituent that are represented by R₁ to R₁₆ in the general formulae (1) to (7) include trimethylsilyl group, triethylsilyl group, t-butyldimethylsilyl group, vinyldimethylsilyl group, propyldimethylsilyl group, etc.
Examples of the arylsilyl group having 8 to 40 carbon atoms and further may have a substituent that are represented by R₁ to R₁₆ in the general formulae (1) to (7) include triphenylsilyl group, tribiphenylsilyl group, di-terphenyl-phenylsilyl group, phenyldimethylsilyl group, t-butyldiphenylsilyl group, etc.
Examples of the aralkylsilyl group having 8 to 40 carbon atoms and further may have a substituent that are represented by R₁ to R₁₆ in the general formulae (1) to (7) include tribenzylsilyl group, benzyldimethylsilyl group, t-butyl dibenzylsilyl group, etc.
Examples of the alkyl germanium group having 3 to 20 carbon atoms and further may have a substituent that are represented by R₁ to R₁₆ in the general formulae (1) to (7) include trimethylgermanium group, triethylgermanium group, t-butyldimethylgermanium group, vinyldimethylgermanium group, propyl dimethylgermanium group, etc.
Examples of the arylgermanium group having 8 to 40 carbon atoms and further may have a substituent that are represented by R₁ to R₁₆ in the general formulae (1) to (7) include triphenylgermanium group, tribiphenylgermanium group, di-terphenyl-phenylgermanium group, phenyldimethylgermanium group, t-butyl diphenylgermanium group, etc.
Examples of the aralkylgermanium group having 8 to 40 carbon atoms and further may have a substituent that are represented by R₁ to R₁₆ in the general formulae (1) to (7) include tribenzylgermanium group, benzildimethylgermanium group, t-butyl dibenzylgermanium group, etc.
The keto aryl group having 7 to 40 carbon atoms and further may have a substituent that are represented by R₁ to R₁₆ in the general formulae (1) to (7) is expressed by -COAr₂, while specific examples of Ar₂ include the same as explained about the foregoing aryl group including the preferable examples.
Examples of the alkyl halide group having 1 to 40 carbon atoms and further may have a substituent that are represented by R₁ to R₁₆ in the general formulae (1) to (7) include those obtained by substituting at least one hydrogen atom by a halogen atom also including the preferable examples.
Examples of the ring structure formed when R₁ to R₁₆ in the general formulae (1) to (7) plurally exist include unsaturated 6-member rings such as benzene rings or so, a saturated or an unsaturated 5-member ring structure, a saturated or an unsaturated 7-member ring structure or so.

Examples of the alkyl group having 1 to 40 carbon atoms represented by Rₐ, R_{b}, R_{c} and R_{d} in the substituted silicon atom expressed by SiRₐR_{b} or the substituted germanium group expressed by GeR_{c}R_{d} each represented by X are the same as the foregoing examples about the above alkyl group and preferable examples are methyl group, ethyl group, propyl group and butyl group. Further, examples of the aryl group having 6 to 20 carbon atoms represented by Rₐ, R_{b}, R_{c} and R_{d} are the same examples as those about the foregoing non-condensed aryl group and preferable examples are phenyl group, p-tolyl group and 4-biphenyl group.

Specific examples of the material for organic EL devices comprising the compounds represented by any one the general formulae (1) to (7) of the present invention include the following compounds, though not limited thereto. Additionally, Me represents a methyl group.

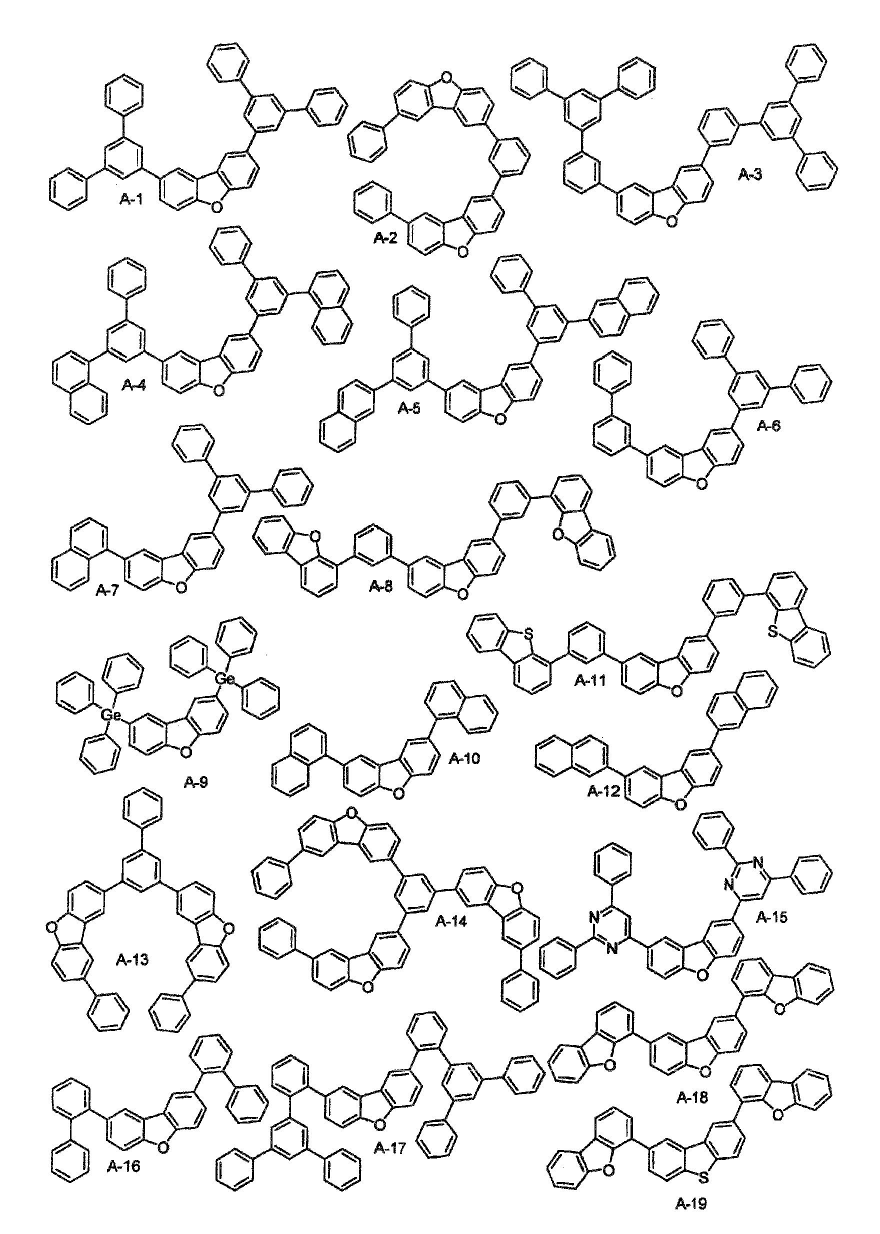

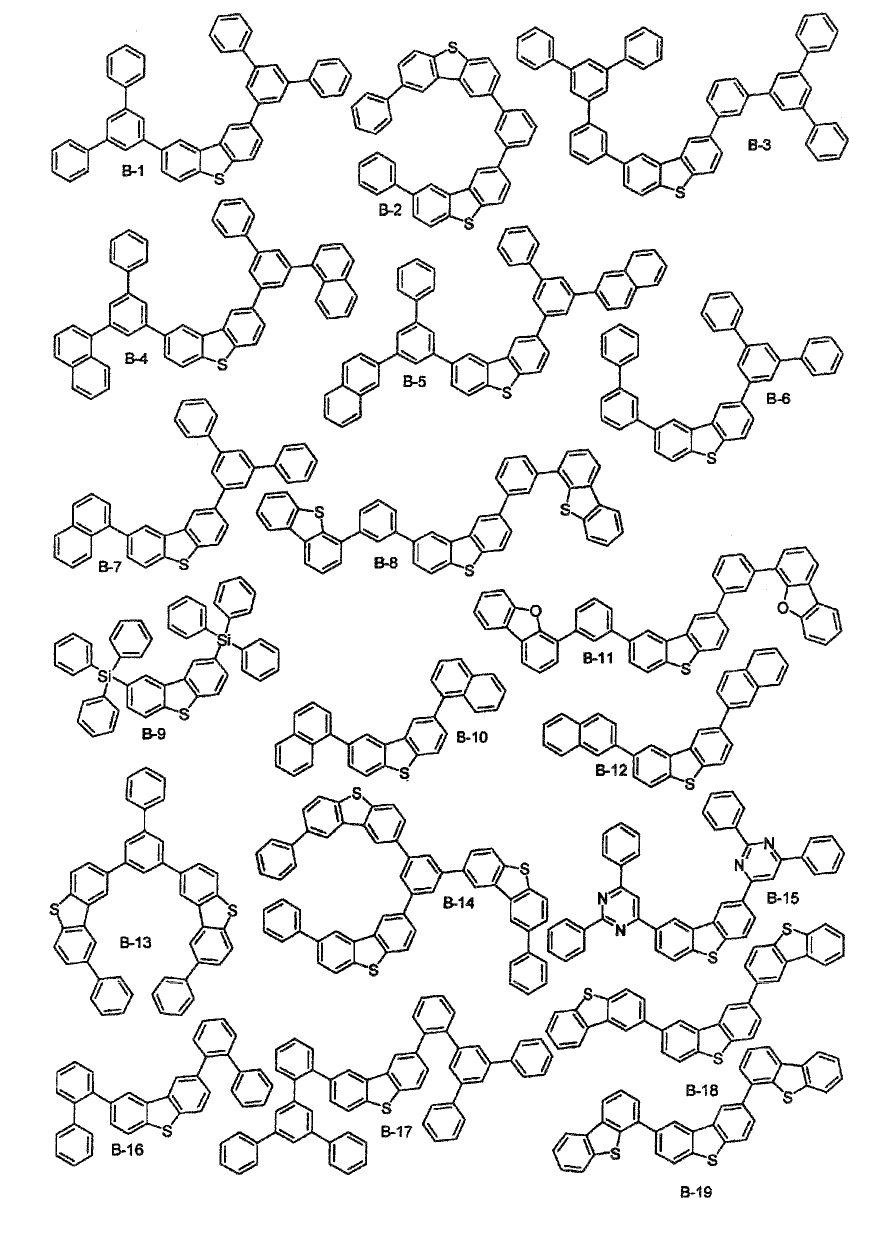

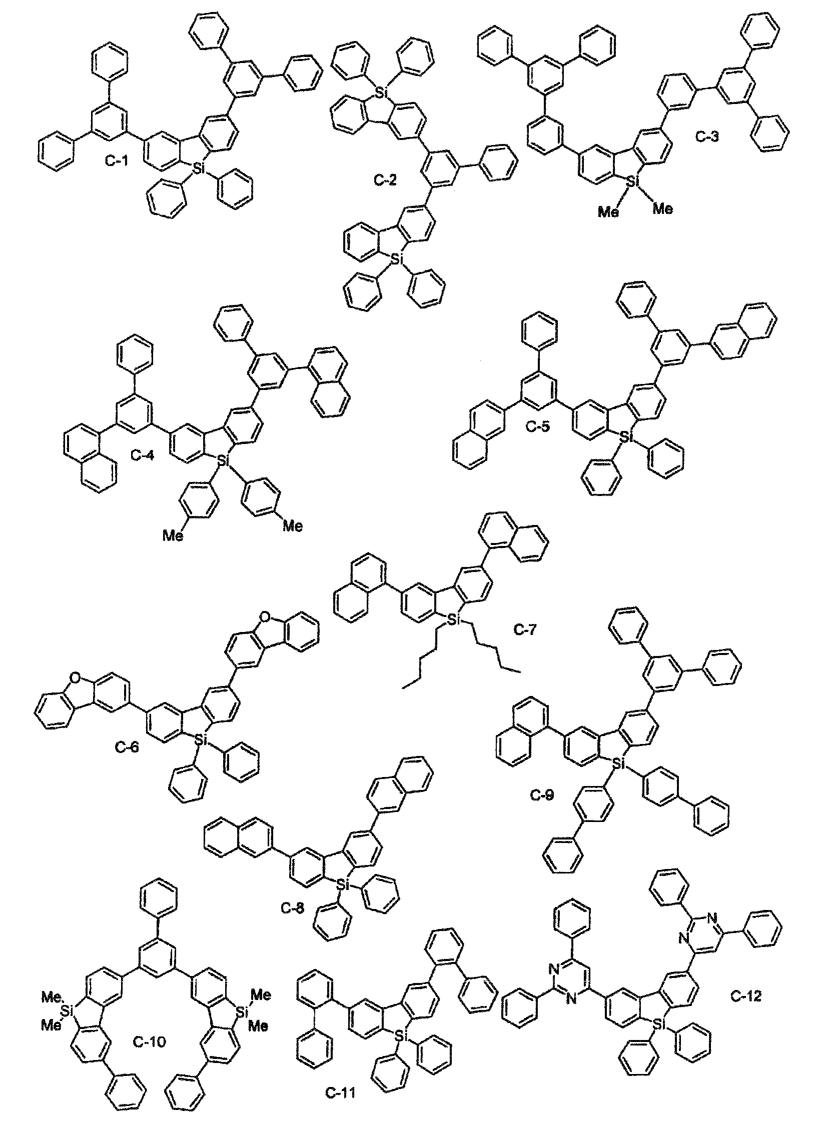

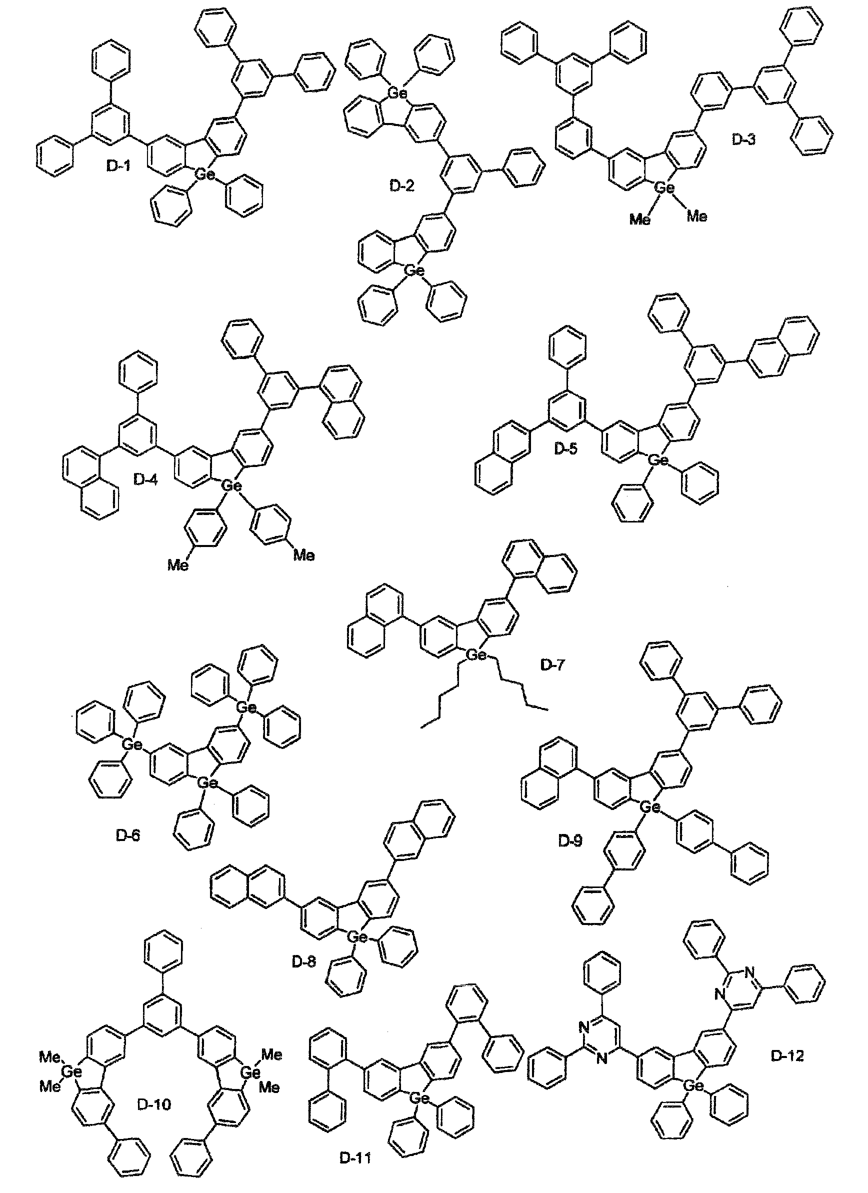

Further, it is preferable that the material for the organic electroluminescence device of the present invention works as a host material in the organic EL device.

The construction of the organic EL device of the present invention will be explained below.
The present invention provides an organic electroluminescence device which comprises at least one organic thin film layer comprising a light emitting layer sandwiched between a pair of electrode consisting of an anode and a cathode, wherein at least one of the organic thin film layer comprises the material for the organic EL device of the present invention.
Typical examples of the construction of the organic EL device of the multi-layer type include an anode / a hole transporting layer (a hole injecting layer) / a light emitting layer / a cathode; an anode / a light emitting layer / an electron transporting layer (an electron injecting layer) / a cathode; an anode / a hole transporting layer (a hole injecting layer) / a light emitting layer / an electron transporting layer (an electron injecting layer) / a cathode; an anode / a hole transporting layer (a hole injecting layer) / a light emitting layer / a hole barrier layer / an electron transporting layer (an electron injecting layer) / a cathode; etc.

The light emitting layer comprises the host material and a phosphorescent material, wherein the host material preferably comprises the foregoing material for the organic EL device.
As the phosphorescent material, iridium complexes, osmium complexes and platinum complexes are preferable, iridium complexes and platinum complexes are more preferable, and iridium complexes in the form of ortho metal are most preferable each since the quantum yield of phosphorescence is great and the external quantum efficiency of the light emitting device can be further increased respectively. As for the further preferable form of ortho metal complex, iridium complexes below are desirable.

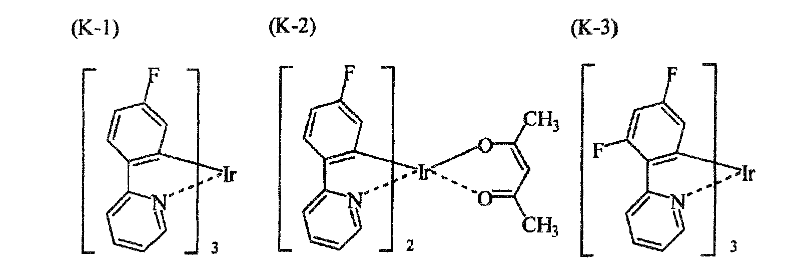

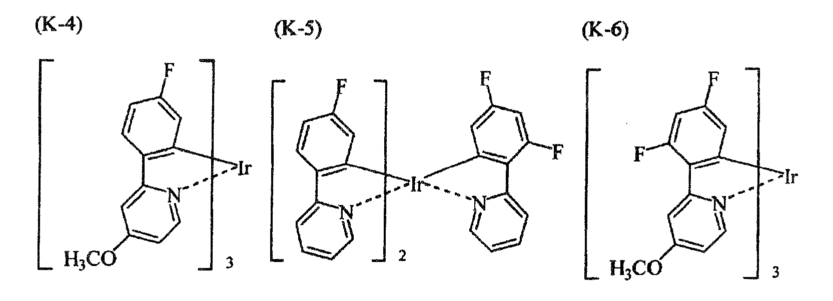

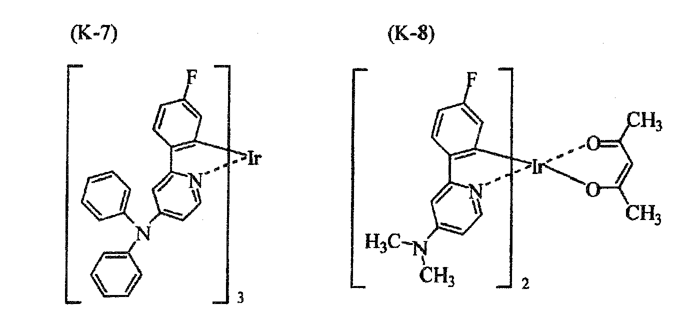

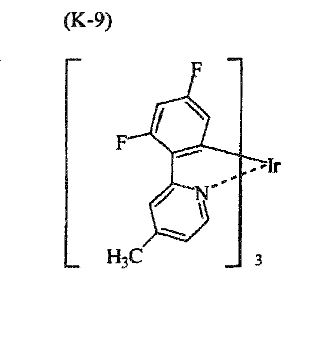

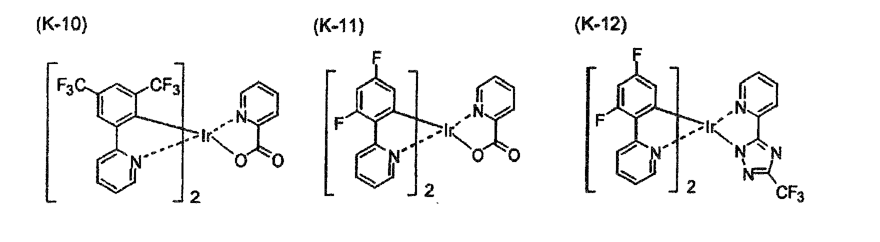

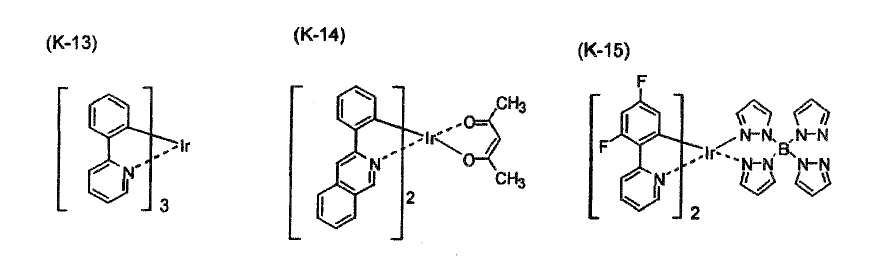

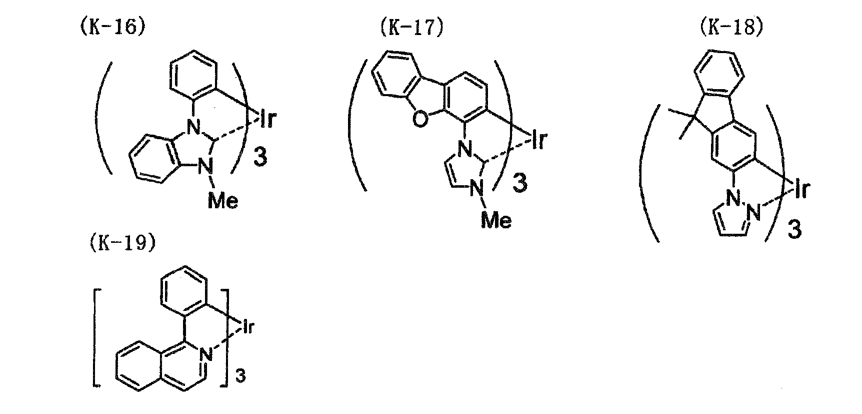

In the organic EL device of the present invention, it is preferable that the light emitting layer comprises the host material and the phosphorescent material and that the phosphorescent material is a light emitting material having a metal-carbene carbon bond.
In the organic EL device of the present invention, it is preferable that the material for the organic electroluminescence device is the host material contained in the light emitting layer of the organic electroluminescence device.
In the organic EL device of the present invention, it is preferable that the material for the organic electroluminescence device is the material contained in the hole transporting layer of the organic electroluminescence device.
In the organic EL device of the present invention, it is preferable that the material for the organic electroluminescence device is the material contained in the hole transporting layer or in the hole barrier layer of the organic electroluminescence device.

In the present invention, it is preferable that the reductive dopant is added in the interfacial region between the cathode and the organic thin film layer of the organic EL device.
Examples of the reductive dopant include at least one compound selected from alkali metals, alkali metal complexes, alkali metal compounds, alkaline earth metals, alkaline earth metal complexes, alkaline earth metal compounds, rare earth metals, rare earth metal complexes and rare earth metal compounds.

Examples of the alkali metal include Na (the work function: 2.36 eV), K (the work function: 2.28 eV), Rb (the work function: 2.16 eV), Cs (the work function: 1.95 eV) and so on; whose work function of 2.9 eV or smaller is particularly preferable. Among those, more preferable alkali metals include K, Rb and Cs, the latter Rb or Cs being farther more preferable and the last Cs being the most preferable.
Examples of the alkaline earth metal include Ca (the work function: 2.9 eV), Sr (the work function: 2.0 to 2.5 eV) and Ba (the work function: 2.52 eV). Alkaline earths metals with a work function of 2.9 eV or smaller are preferable.
Examples of the rare earth metal include Sc, Y, Ce, Tb and Yb. Rare earths metals with a work function of 2.9 eV or smaller are preferable.
Those alkaline metals have particularly high reducing capability, and only an addition of relatively small amount of them into an electron injection region enables to expect both improvement of luminance and lifetime extension of the organic EL device.

Examples of the alkali metal compound described above include alkali metal oxides such as Li₂O, Cs₂O and K₂O and alkali metal halides such as LiF, NaF, CsF and KF. Among these compounds, alkali metal oxides and alkali metal fluorides such as LiF, Li₂O and NaF are preferable.
Examples of the alkaline earth metal compound described above include BaO, SrO, CaO and mixtures thereof such as BaₓSr₁₋ₓO (0<x<1) and BaₓCa₁₋ₓO (0<x<1), while BaO, SrO and CaO are preferable.
Examples of the rare earth metal compound described above include YbF₃, ScF₃, ScO₃, Y₂O₃, Ce₂O₃, GdF₃ and TbF₃. Among these compounds, YbF₃, ScF₃ and TbF₃ are preferable.

The alkali metal complex, the alkaline earth metal complex and the rare earth metal complex are not particularly limited as long as the complexes contain at least one of the alkali metal ions, the alkaline earth metal ions and rare earth metal ions, respectively, as the metal ion. As a ligand, quinolinol, benzoquinolinol, acridinol, phenanthridinol, hydroxyphenyloxazole, hydroxyphenylthiazole, hydroxydiaryloxadiazoles, hydroxydiarylthiadiazoles, hydroxyphenylpyridine, hydroxyphenyl-benzimidazole, hydroxybenzotriazole, hydroxyfulvorane, bipyridyl, phenanthroline, phthalocyanine, porphyrin, cyclopentadiene, β-diketones, azomethines and derivatives of these compounds are preferable. However, the ligand is not limited to the ligands described above.

As for the addition form of the reductive dopant, it is preferable that the reductive dopant is added in a manner such that a layer or islands are formed in the interfacial region described above. As the process for adding the reductive dopant, it is preferable that an organic material which is the light emitting material or the electron injecting material forming the interfacial region is vaporized while the reductive dopant is simultaneously vapor deposited in accordance with the resistance heating deposition method so that the reductive dopant is dispersed in the organic material. The concentration of the dispersion expressed as the ratio of the amounts by mole of the organic substance to the reductive dopant is in the range of 100:1 to 1:100 and preferably in the range of 5:1 to 1:5.
When the reductive dopant is added to form a layer, the reductive dopant alone is vapor deposited in accordance with the resistance heating deposition method to form a layer preferably having a thickness of 0.1 to 15 nm after a layer of the organic material such as the light emitting material and the electron injecting material is formed as the interfacial region. When the reductive dopant is added to form islands, the reductive dopant alone is vapor deposited in accordance with the resistance heating deposition method to form islands preferably having a thickness of 0.1 to 15 nm after islands of the organic material such as the light emitting material and the electron injecting material were formed as the interfacial region.
It is preferable that the relative amounts by mole of the main component and the reductive dopant in the electron injecting layer of the organic EL device of the present invention is in the range of 5:1 to 1:5 and more preferably in the range of 2:1 to 1:2.

It is preferable that the organic EL device of the present invention has an electron injecting layer between the light emitting layer and the cathode and that the electron injecting layer essentially comprises a nitrogen atom-containing ring derivative.
An aromatic heterocyclic compound having at least one hetero atom in its molecular is preferably employed as the electron transporting material used in the electron injecting layer, and a nitrogen atom-containing ring derivative being particularly preferable.

For example, a specific compound represented by a following general formula (A) is preferable as the nitrogen atom-containing ring derivative.

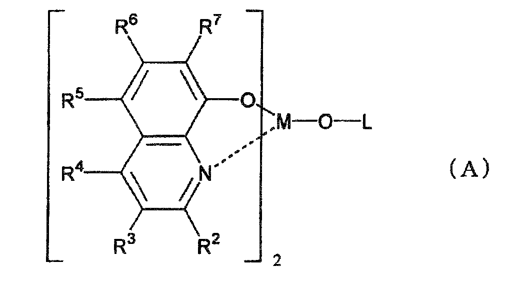

wherein R² to R⁷ each independently represents a hydrogen atom, a halogen atom, an oxy group, an amino group or a hydrocarbon group having 1 to 40 carbon atoms, that may be substituted respectively.
Specific examples of the halogen atom are the same as the foregoing description. Further, examples of the above amino group which may be substituted are the same as the description about the foregoing alkylamino group, the foregoing arylamino group and the foregoing aralkylamino group.
Examples of the hydrocarbon group having 1 to 40 carbon atoms include a substituted or unsubstituted alkyl group, a substituted or unsubstituted alkenyl group, a substituted or unsubstituted cycloalkyl group, a substituted or unsubstituted alkoxy group, a substituted or unsubstituted aryl group, a substituted or unsubstituted heterocyclic group, a substituted or unsubstituted aralkyl group, a substituted or unsubstituted aryloxy group, a substituted or unsubstituted alkoxycarbonyl group, etc. Examples of the alkyl group, the alkenyl group, the cycloalkyl group, the alkoxy group, aryl group, the heterocyclic group, the aralkyl group and the aryloxy group are the same as the foregoing description. The alkoxycarbonyl group is expressed by -COOY', while examples of Y' are the same as the examples of the above alkyl group.

M in the general formula (A) represents aluminum (Al), gallium (Ga) or indium (In), preferably In.
L in the general formula (A) is a group expressed by a following general formula (A') or a following general formula (A"). 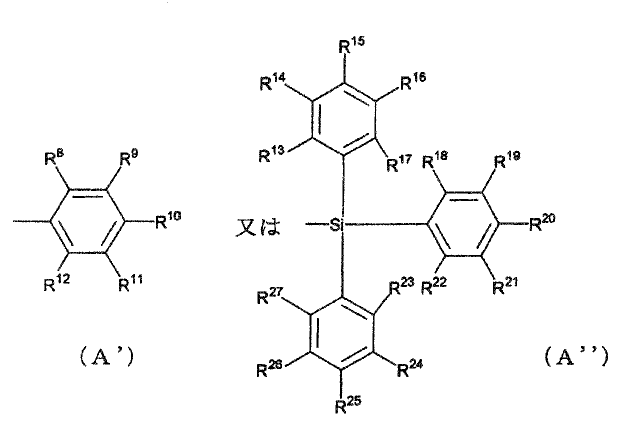 wherein R⁸ to R¹² each independently represents a hydrogen atom or a substituted or unsubstituted hydrocarbon group having 1 to 40 carbon atoms, while an adjacent group may form a ring structure between each other respectively. Further, R¹³ to R²⁷ each independently represents a hydrogen atom or a substituted or unsubstituted hydrocarbon group having 1 to 40 carbon atoms, while an adjacent group may form a ring structure between each other respectively.

Examples of the hydrocarbon group having 1 to 40 carbon atoms represented by R⁸ to R¹² in the general formula (A') and by R¹³ to R²⁷ in the general formula (A") are the same specific examples as the foregoing R² to R⁷.
Furthermore, examples of a bivalent group made by bonding the adjacent groups among the above R⁸ to R¹² and R¹³ to R²⁷ to form a ring structure include tetramethylene group, pentamethylene group, hexamethylene group, diphenyl-methane-2,2'-diyl group, diphenylethane-3,3'-diyl group, diphenylpropane-4, 4'-diyl group, etc.

Specific examples of the metal chelate complex having a nitrogen atom represented by the general formula (A) include the following compounds, though not limited thereto.

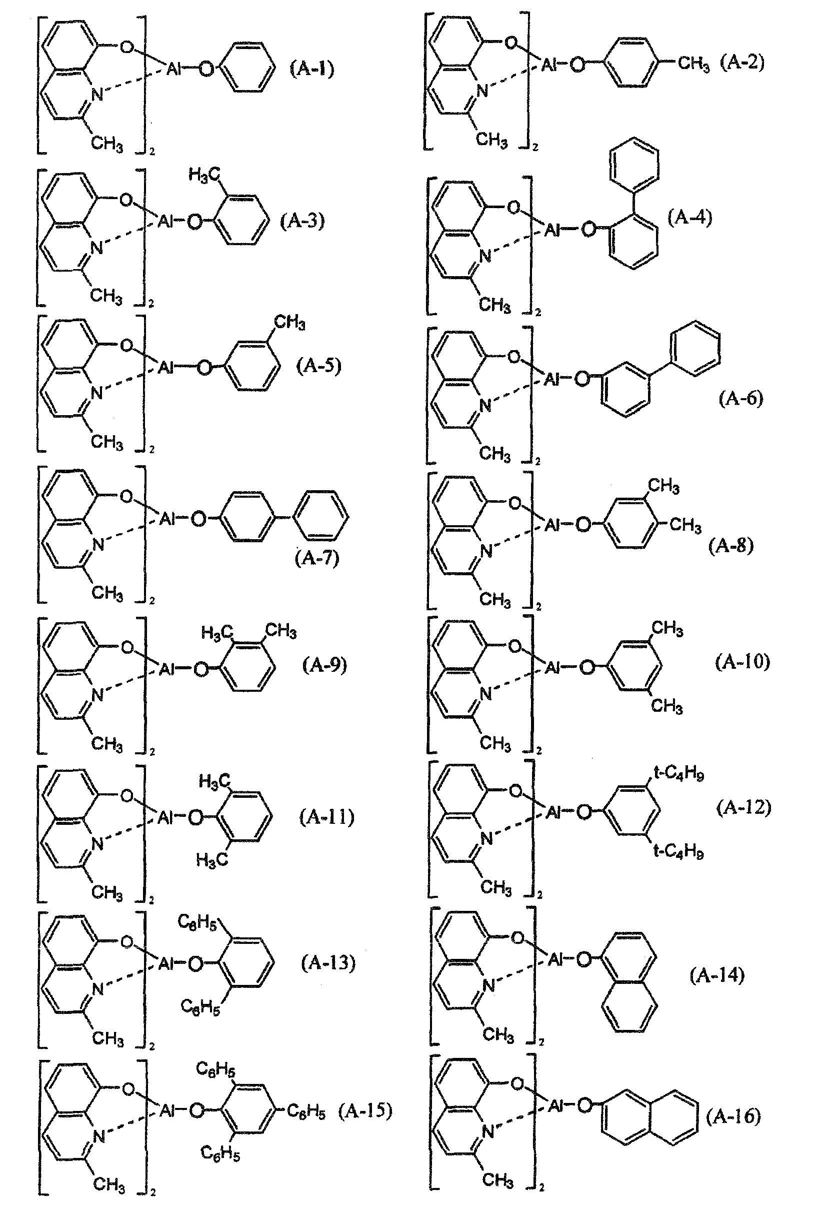

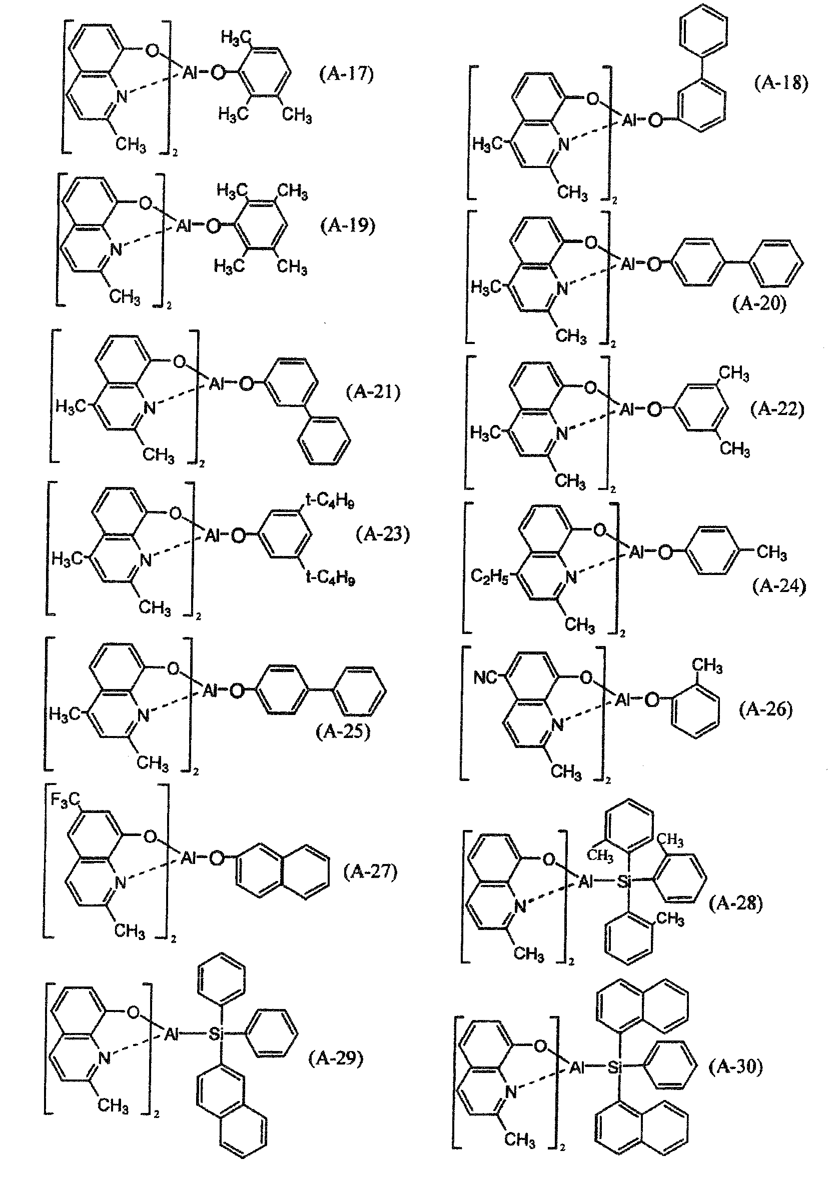

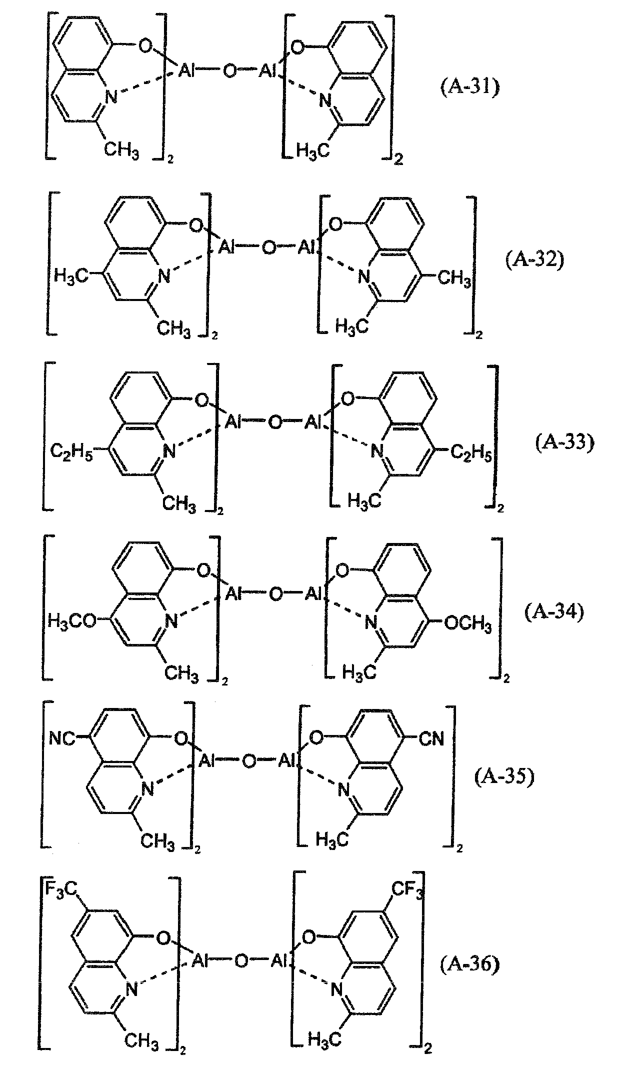

With regard to the nitrogen atom-containing ring derivative as an essential component of the electron injecting layer, it is preferably a 5-member ring derivative having a nitrogen atom and examples of the 5-member ring include imidazole ring, triazole ring, tetrazole ring, oxadiazole ring, thiadiazole ring, oxatriazole ring, thiatriazole ring, etc. Examples of the 5-member ring derivative having a nitrogen atom include benzimidazole ring, benztriazole ring, pyridinoimidazole ring, pyrimidinoimidazole ring and pyridazinoimidazole ring; while the 5-member ring derivative having a nitrogen atom being particularly preferable to be represented by a following general formula (B):

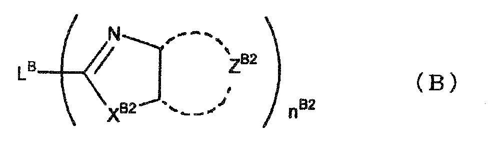

In the general formula (B), L^{B} represents a bonding group with bivalent or more, examples include carbon atom, silicon atom, nitrogen atom, boron atom, oxygen atom, sulfur atom, metals (for example, barium, beryllium), aromatic hydrocarbon ring, aromatic heterocycles and so on. Among those, carbon atom, nitrogen atom, silicon atom, boron atom, oxygen atom, sulfur atom, aryl group or aromatic heterocyclic group is preferable; and carbon atom, silicon atom, aryl group or aromatic heterocyclic group is further preferable.
The aryl group and the aromatic heterocyclic group represented by L^{B} may have a substituent, and preferable examples of the substituent are alkyl group, alkenyl group, alkynyl group, aryl group, amino group, alkoxy group, aryloxy group, acyl group, alkoxycarbonyl group, aryloxycarbonyl group, acyloxy group, acylamino group, alkoxycarbonylamino group, aryloxy carbonylamino group, sulfonylamino group, sulfamoyl group, carbamoyl group, alkylthio group, arylthio group, sulfonyl group, halogen atom, cyano group and aromatic heterocycle group; more preferable examples are alkyl group, aryl group, alkoxy group, aryloxy group, halogen atom, cyano group and aromatic heterocycle group; furthermore preferable examples are alkyl group, aryl group, alkoxy group, aryloxy group and aromatic heterocycle group; and particularly preferable examples are alkyl group, aryl group, alkoxy group and aromatic heterocycle group.
Specific examples of L^{B} are as follows:

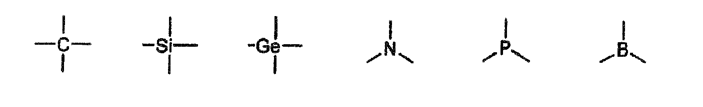

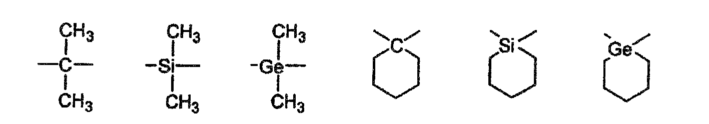

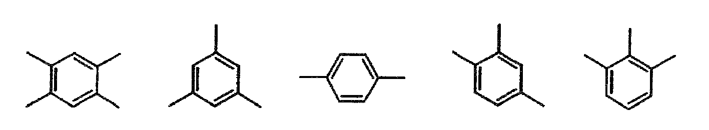

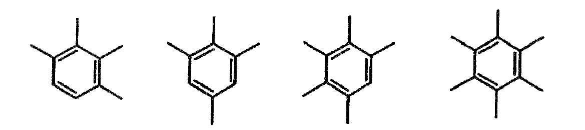

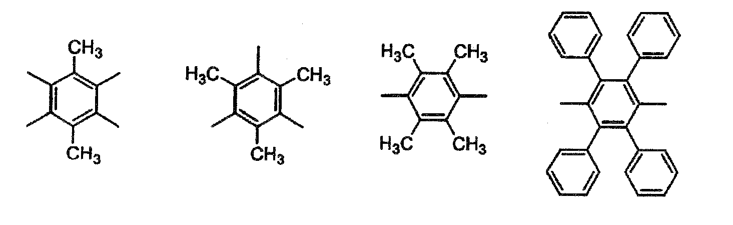

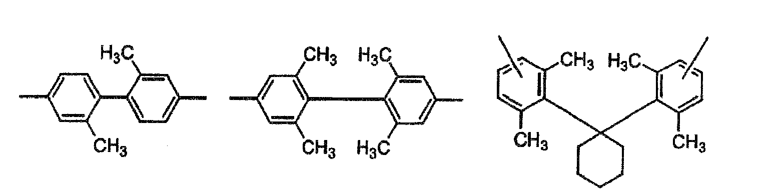

X^{B2} in the general formula (B) represents -O-, -S- or =N-R^{B2}. R^{B2} represents a hydrogen atom, an aliphatic hydrocarbon group, an aryl group or a heterocyclic group.
Examples of the aliphatic hydrocarbon group represented by R^{B2} include linear, branched or cyclic alkyl group; linear, branched or cyclic alkenyl group; and linear, branched or cyclic alkynyl group. The linear, branched or cyclic alkyl group has preferably 1 to 20 carbon atoms, more preferably 1 to 12 carbon atoms, particularly preferably 1 to 8 carbon atoms and examples include methyl group, ethyl group, iso propyl group, tert-butyl group, n-octyl group, n-decyl group, n-hexadecyl group, cyclopropyl group, cyclopentyl group, cyclohexyl group, etc. The linear, branched or cyclic alkenyl group has preferably 2 to 20 carbon atoms, more preferably 2 to 12 carbon atoms, particularly preferably 2 to 8 carbon atoms and examples include vinyl group, aryl group, 2-butenyl group, 3-pentenyl group, etc. The linear, branched or cyclic alkynyl group has preferably 2 to 20 carbon atoms, more preferably 2 to 12 carbon atoms, particularly preferably 2 to 8 carbon atoms and examples include propargyl group, 3-pentinyl group, etc. Among those, the linear, branched or cyclic alkyl group is the most preferable.

The aryl group represented by R^{B2} consists of single ring or condensed ring, having preferably 6 to 30 carbon atoms, more preferably 6 to 20 carbon atoms, further more preferably 6 to 12 carbon atoms and examples include phenyl, 2-methylphenyl, 3-methylphenyl, 4-methylphenyl, 2-methoxyphenyl, 3-trifluoromethylphenyl, pentafluorophenyl, 1-naphthyl, 2-naphthyl, etc.

The heterocyclic group represented by R^{B2} consists of single ring or condensed ring, having preferably 1 to 20 carbon atoms, more preferably 1 to 12 carbon atoms, further more preferably 1 to 10 carbon atoms and specifically, it is preferable to be an aromatic heterocyclic group having at least one selected from a group consisting of a nitrogen atom, an oxygen atom, a sulfur atom and a selenium atom. Examples of the heterocyclic group include pyrrolidine, piperidine, piperazine, morpholine, thiophene, selenophene, furan, pyrrole, imidazole, pyrazole, pyridine, pyrazine, pyridazine, pyrimidine, triazole, triazine, indole, indazole, purine, thiazoline, thiazole, thiadiazole, oxazoline, oxazole, oxadiazole, chinoline, isoquinoline, phthalazine, naphthyridine, quinoxaline, quinazoline, quinoliine, pteridine, acridine, phenanthroline, phenazine, tetrazole, benzimidazole, benzoxazole, benzothiazole, benztriazole, tetrazaindene, carbazole, azepin, etc.; while furan, thiophene, pyridine, pyrazine, pyrimidine, pyridazine, triazine, quinoline, phthalazine, naphthyridine, quinoxaline and quinazoline are preferable; furan, thiophene, pyridine and quinoline are more preferable; and quinoline is further more preferable.

The aliphatic hydrocarbon group, the aryl group and the heterocyclic group represented by R^{B2} may have a substituent whose examples are the same as the above examples of the substituent of the foregoing L^{B} including the preferable examples.
The aliphatic hydrocarbon group, the aryl group and the heterocyclic group are preferable as R^{B2}, the aliphatic hydrocarbon group having desirably 6 to 30 carbon atoms, more desirably 6 to 20 carbon atoms and further more desirably 6 to 12 carbon atoms or the aryl group are more preferable and the aliphatic hydrocarbon group having desirably 1 to 20 carbon atoms, more desirably 1 to 12 carbon atoms and further more desirably 2 to 10 carbon atoms is further more preferable.
In the general formula (B), X^{B2} is preferably -O- or =N-R^{B2} and particularly preferably =N-R^{B2}.

In the general formula (B), Z^{B2} represents necessary atom group in order to form aromatic ring. The aromatic ring formed by Z^{B2} may be any of an aromatic hydrocarbon ring or an aromatic heterocycles; examples include benzene ring, pyridine ring, pyrazine ring, pyrimidine ring, pyridazine ring, triazine ring, pyrrole ring, furan ring, thiophene ring, selenophene ring, tellurophene ring, imidazole ring, thiazole ring, selenazole ring, tellurazole ring, thiadiazole ring, oxadiazole ring, pyrazole ring, etc.; while benzene ring, pyridine ring, pyrazine ring, pyrimidine ring and pyridazine ring are preferable; benzene ring, pyridine ring and pyrazine ring are more preferable; benzene ring and pyridine ring are further more preferable; and pyridine ring is particularly preferable.

The aromatic ring formed by Z^{B2} may form a condensed ring with other ring or may have a substituent. Preferable substituent for Z^{B2} are alkyl group, alkenyl group, alkynyl group, aryl group, amino group, alkoxy group, aryloxy group, acyl group, alkoxycarbonyl group, aryloxy carbonyl group, acyloxy group, acylamino group, alkoxycarbonylamino group, aryloxycarbonylamino group, sulfonylamino group, sulfamoyl group, carbamoyl group, alkylthio group, arylthio group, sulfonyl group, halogen atom, cyano group and heterocyclic group; more preferable substituent for Z^{B2} are alkyl group, aryl group, alkoxy group, aryloxy group, halogen atom, cyano group and heterocyclic group; further more preferable substituent for Z^{B2} are alkyl group, aryl group, alkoxy group, aryloxy group and heterocyclic group; particularly preferable substituent for Z^{B2} are alkyl group, aryl group, alkoxy group and heterocyclic group.
In the general formula (B), n^{B2} represents an integer of 1 to 4, preferably an integer of 2 or 3.

Among the 5-member ring derivative having a nitrogen atom represented by the foregoing general formula (B), further preferable derivatives are expressed by a following general formula (B'):

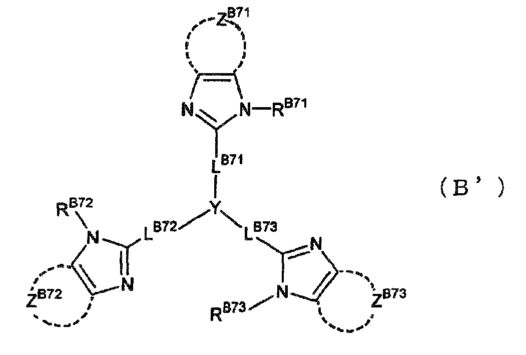

In the general formula (B'), R^{B71}, R^{B72} and R^{B73} each represents the same as R^{B2} in the general formula (B), wherein the preferable examples are also the same.
In the general formula (B'), Z^{B71}, Z^{B72} and Z^{B73} each represents the same as Z^{B2} in the general formula (B), wherein the preferable examples are also the same.
In general formula (B'), L^{B71}, L^{B72} and L^{B73} each represents a bonding group of bivalent described as the examples of L^{B} in the general formula (B); preferably a single bond, a bivalent aromatic hydrocarbon ring group, a bivalent aromatic heterocyclic group; and the bonding group formed by combining those; more preferably a single bond. L^{B71}, L^{B72} and L^{B73} may have a substituent, whose examples are the same as described about the substituent for L^{B} in the general formula (B) including the preferable examples.
Y represents a nitrogen atom, a 1,3,5-benzenetriyl group or a 2,4,6-triazinetriyl group. The 1,3,5-benzenetriyl group may have substituent at 2, 4 and 6-positions, examples of the substituent include alkyl group, aromatic hydrocarbon ring group, halogen atom, etc.

Specific examples of the derivatives of 5-member ring having a nitrogen atom represented by the general formula (B) or the general formula (B') include the following compounds, though not limited thereto.

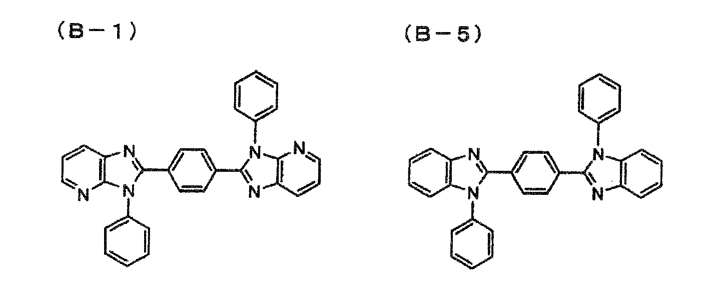

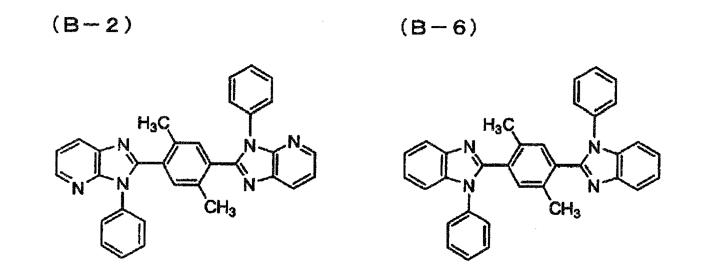

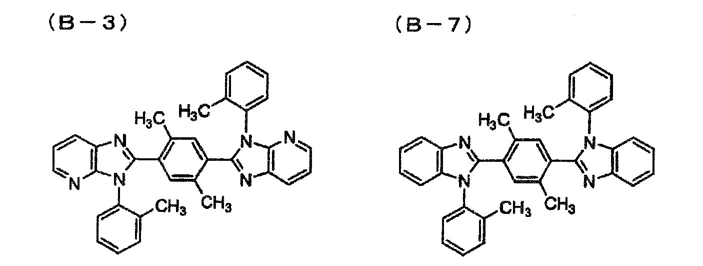

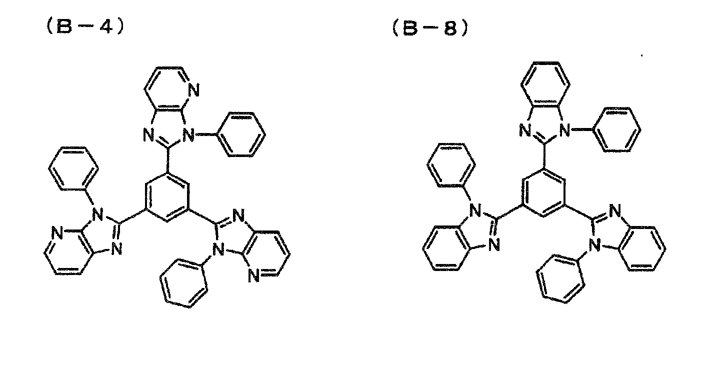

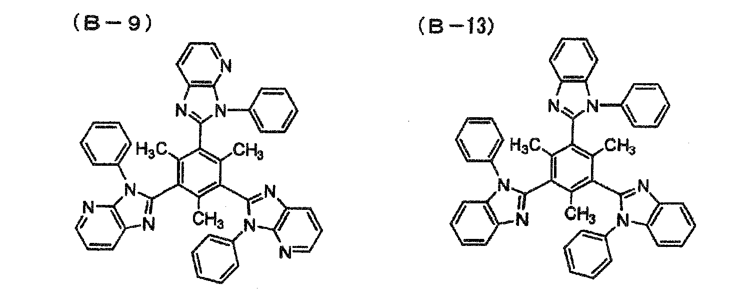

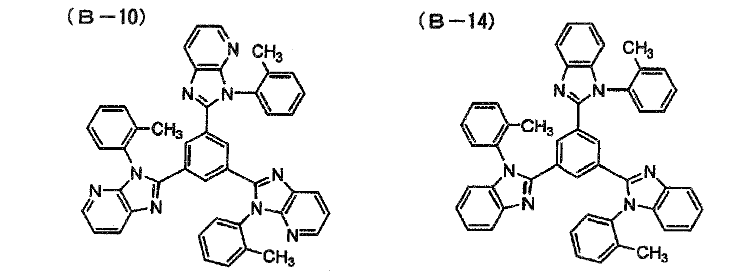

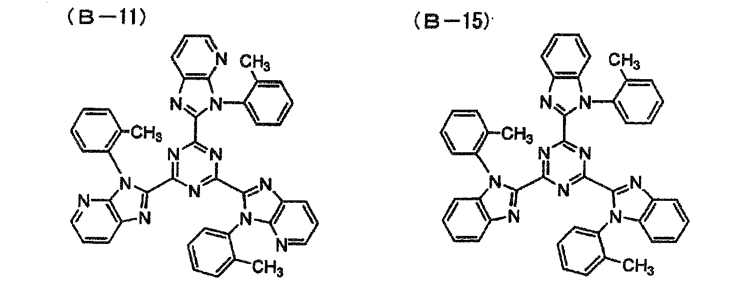

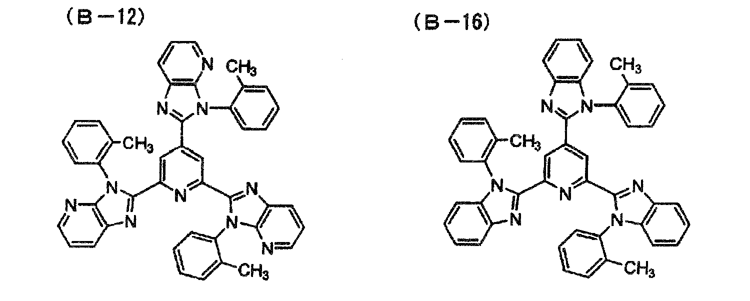

Regarding with a compound constituting the electron injecting layer or the electron transporting layer, the compound having a structure made by combining an electron lacking 5 or 6-member ring skeleton having a nitrogen atom with any of a substituted or unsubstituted indole skeleton, a substituted or unsubstituted carbazole skeleton and a substituted or unsubstituted azacarbazole skeleton will be employable. Preferable examples of the electron lacking 5 or 6-member ring skeleton include pyridine skeleton, pyrimidine skeleton, pyrazine skeleton, triazine skeleton, triazole skeleton, oxadiazole skeleton, pyrazole skeleton, imidazole skeleton, quinoxaline skeleton, pyrrole skeleton and a molecular skeletons generated by condensing those respectively, such as benzimidazole, imidazopyridine, etc. Among those, a preferable combination is obtained by combining at least one selected from a group consisting of pyridine skeleton, pyrimidine skeleton, pyrazine skeleton and triazine skeleton with at least one selected from a group consisting of carbazole skeleton, indole skeleton, azacarbazole skeleton and quinoxaline skeleton. The above-mentioned skeleton may be substituted or may be unsubstituted.
Specific examples of the electron transportable compound will be shown below:

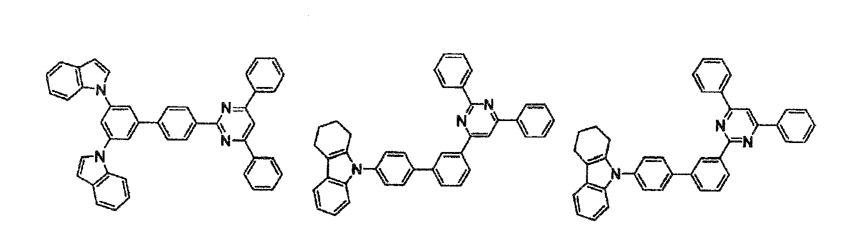

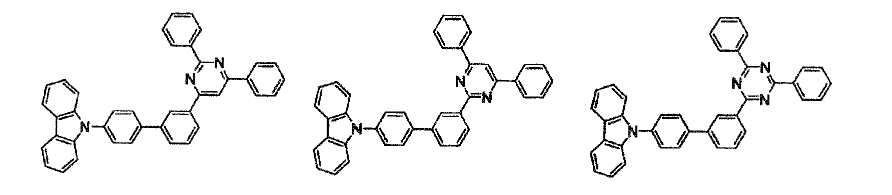

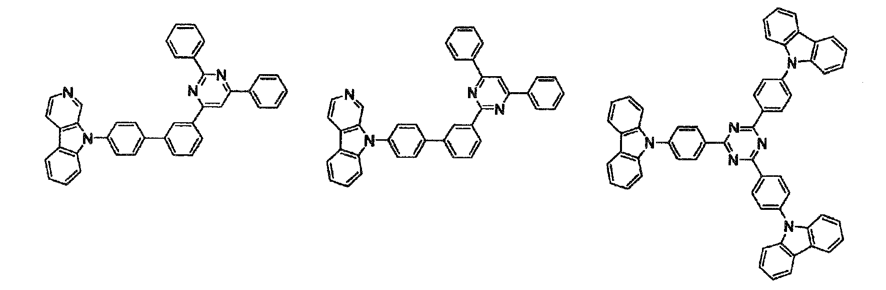

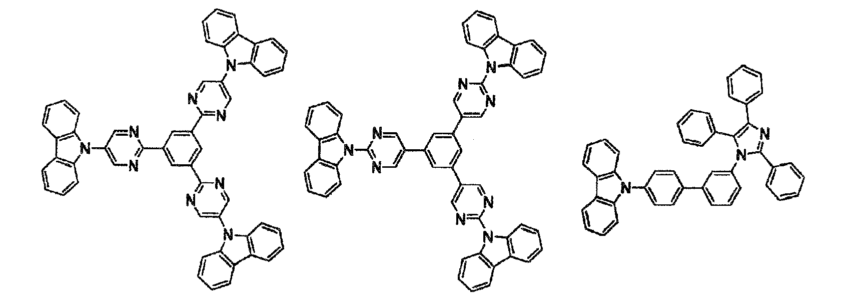

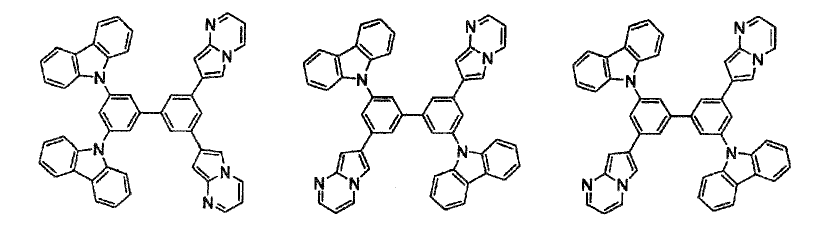

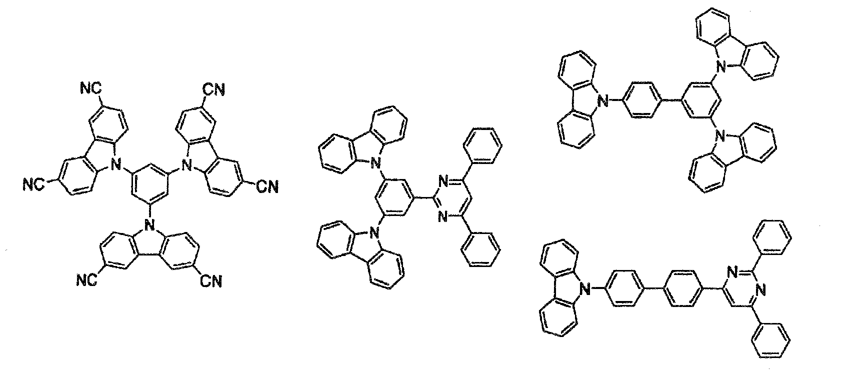

The electron injecting layer and the electron transporting layer may be composed of single layer comprising one or more kind of these materials or may be laminated with an electron injecting layer or an electron transporting layer comprising another kind of compound. It is preferable that the materials are π -electron lacking heterocyclic group having a nitrogen atom.

Further in the organic EL device of the present invention, it is preferable to employ an inorganic compound such as an insulating material or a semiconductor for an electron injecting layer. The electron injecting layer employing an insulating material or a semiconductor effectively prevents leak in the electric current and improves the electron injecting capability.
With regard to the electric insulator, an employment of at least one or more kinds of metal compound selected from the group consisting of alkaline metal chalcogenide, alkaline earth metal chalcogenide, halide of alkaline metal and halide of alkaline earth metal is preferable. It is preferable that the electron injecting layer is constituted with the above alkali metal chalcogenide since the electron injecting property can be improved. Preferable examples of the alkali metal chalcogenide include Li₂O, K₂O, Na₂S and Na₂Se. Preferable examples of the alkaline earth metal chalcogenide include CaO, BaO, SrO, BeO, BaS and CaSe. Preferable examples of the alkali metal halide include LiF, NaF, KF, LiCl, KCl and NaCl. Preferable examples of the alkaline earth metal halide include fluorides such as CaF₂, BaF₂, SrF₂, MgF₂ and BeF₂ and halides other than the fluorides.
Examples of the semiconductor constituting the electron injecting layer include oxides, nitrides and nitriding oxides containing at least one element selected from Ba, Ca, Sr, Yb, Al, Ga, In, Li, Na, Cd, Mg, Si, Ta, Sb and Zn, which are used singly or in combination of two or more. It is preferable that the inorganic compound constituting the electron transporting layer is in the form of a fine crystalline or amorphous insulating thin film. When the electron transporting layer is constituted with the above insulating thin film, a more uniform thin film can be formed and defective pixels such as dark spots can be decreased. Examples of the inorganic compound include the alkali metal chalcogenides, the alkaline earth metal chalcogenides, the alkali metal halides and the alkaline earth metal halides which are described above.
In the present invention, a reductive dopant may be added in the electron injecting or transporting layer.

In the present invention, the anode in the organic EL device covers a role of injecting holes into a hole transport layer or a light emitting layer, and it is effective that the anode has a work function of 4.5 eV or greater. Specific examples of the material for the anode include indium tin oxide alloy (ITO), tin oxide (NESA), gold, silver, platinum, copper, etc. With regard to the cathode, its material preferably has a small work function with the aim of injecting electrons into an electron transporting layer or into a light emitting layer. Although materials for the cathode of the organic EL device are not particularly specified, examples include indium, aluminum, magnesium, magnesium-indium alloy, magnesium-aluminum alloy, aluminum-lithium alloy, aluminum-scandium-lithium alloy, magnesium-silver alloy, etc.

The process for forming the layers in the organic EL device of the present invention is not particularly limited. A conventional process such as the vacuum vapor deposition process and the spin coating process can be used. The organic thin film layer used in the organic EL device of the present invention can be formed in accordance with the vacuum vapor deposition process, the molecular beam epitaxy process (the MBE process) or, using a solution prepared by dissolving the compound represented by the foregoing general formulae (1) to (3) into a solvent, in accordance with a conventional coating process such as the dipping process, the spin coating process, the casting process, the bar coating process and the roller coating process.
The thickness of each layer in the organic thin film layer in the organic EL device of the present invention is not particularly limited. In general, an excessively thin layer tends to have defects such as pin holes, and an excessively thick layer requires a high applied voltage resultantly decreasing the efficiency. Therefore, a thickness within the range of several nanometers to 1 µm is preferable.

### EXAMPLES

The present invention shall be explained below in further details with reference to examples.

### Synthesis Example 1

The route for synthesis of Compound (B-1) is shown in the following. 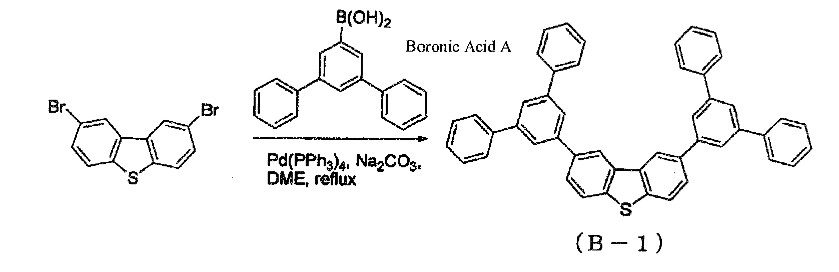 Under the atmosphere of argon gas, 2,8-dibromodibenzothiophene in an amount of 1.31 g (3.83 mmol), boronic acid A in an amount of 2.50 g (9.12 mmol) and tetrakis(triphenylphosphine)palladium in an amount of 0.527 g (0.456 mmol) were placed into a three neck flask with a capacity of 300 milliliter, and the air inside the flask was replaced with argon gas. Adding 1,2-dimethoxyethane in an amount of 27.4 milliliter and 2.0 M sodium carbonate aqueous solution in an amount of 13.7 milliliter (27.4 mmol) into the flask, the resultant solution was refluxed by heating for 9 hours under the atmosphere of argon gas. Water in an amount of 100 milliliter and methylene chloride in an amount of 100 milliliter were added to the resultant reaction solution, and an organic layer was separated, followed by drying with the use of anhydride magnesium sulfide. After concentrating the dried mixture under reduced pressure by means of an evaporator, a resultant solid was purified by means of silicagel column chromatography (dissolution solvent: methylene chloride / hexane= 1/2) and as a result, 2.09 g (3.26 mmol; the yield: 85 %) of Compound (B-1) was obtained. It was confirmed in accordance with 90 MHz ¹H-NMR and Field Desorption Mass Spectrometry (FD-MS) that the obtained crystals were the aimed Compound (B-1). The result of the measurement in accordance with FD-MS is shown as the following:
FD-MS: calcd for C₄₈H₃₀S₂=640, found: m/ z= 640 (M⁺, 100)

### Synthesis Example 2

The route for synthesis of Compound (A-1) is shown in the following. 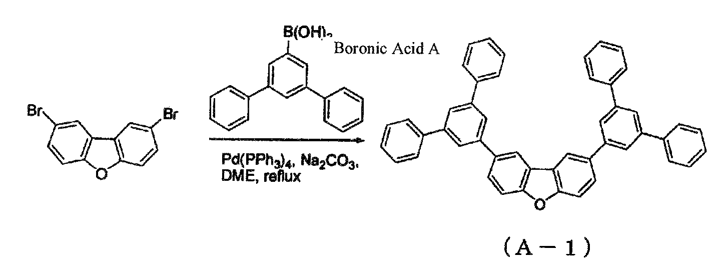 Under the atmosphere of argon gas, 2,8-dibromodibenzofuran in an amount of 2.53 g (7.76 mmol), boronic acid A in an amount of 5.07 g (18.5 mmol) and tetrakis(triphenylphosphine)palladium in an amount of 1.07 g (0.925 mmol) were placed into a three neck flask with a capacity of 300 milliliter, and the air inside the flask was replaced with argon gas. Adding 1,2-dimethoxyethane in an amount of 55.5 milliliter and 2.0 M sodium carbonate aqueous solution in an amount of 27.8 milliliter (55.5 mmol) into the flask, the resultant solution was refluxed by heating for 9 hours under the atmosphere of argon gas. Water in an amount of 100 milliliter and methylene chloride in an amount of 100 milliliter were added to the resultant reaction solution, and an organic layer was separated, followed by drying with the use of anhydride magnesium sulfide. After concentrating the dried mixture under reduced pressure by means of an evaporator, a resultant solid was purified by means of silicagel column chromatography (dissolution solvent: methylene chloride / hexane= 1/2) and as a result, 3.83 g (6.13 mmol; the yield: 79 %) of Compound (A-1) was obtained. The compound was confirmed as the aimed Compound (A-1) from the result in accordance with Field Desorption Mass Spectrum (FD-MS) analysis. The result of the measurement in accordance with FD-MS is shown as the following:
FD-MS: calcd for C₄₈H₃₀O₂= 624, found: m/ z =624 (M⁺, 100)

### Synthesis Example 3

The route for synthesis of Compound (B-16) is shown in the following. 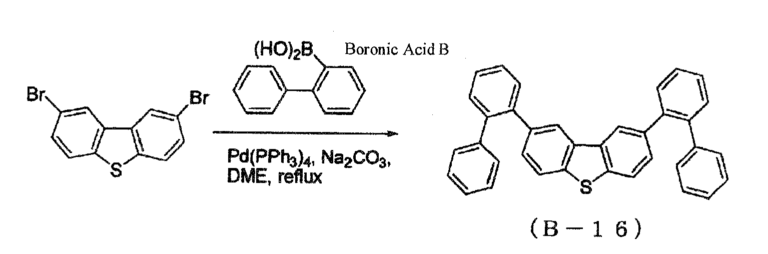 Under the atmosphere of argon gas, 2,8-dibromodibenzothiophene in an amount of 2.15 g (6.29 mmol), boronic acid B in an amount of 2.97 g (15.0 mmol) and tetrakis(triphenylphosphine)palladium in an amount of 0.867 g (0.750 mmol) were placed into a three neck flask with a capacity of 300 milliliter, and the air inside the flask was replaced with argon gas. Adding 1,2-dimethoxyethane in an amount of 45.0 milliliter and 2.0 M sodium carbonate aqueous solution in an amount of 22.5 milliliter (45.0 mmol) into the flask, the resultant solution was refluxed by heating for 10 hours under the atmosphere of argon gas. Water in an amount of 100 milliliter and methylene chloride in an amount of 100 milliliter were added to the resultant reaction solution, and an organic layer was separated, followed by drying with the use of anhydride magnesium sulfide. After concentrating the dried mixture under reduced pressure by means of an evaporator, a resultant solid was purified by means of silicagel column chromatography (dissolution solvent: methylene chloride / hexane= 1/2) and as a result, 1.84 g (3.77 mmol; the yield: 60 %) of Compound (B-16) was obtained. The compound was confirmed as the aimed Compound (B-16) from the result in accordance with Field Desorption Mass Spectrum (FD-MS) analysis. The result of the measurement in accordance with FD-MS is shown as the following:
FD-MS: calcd for C₃₆H₂₄S= 488, found: m/z= 488 (M⁺, 100)

### Synthesis Example 4

The route for synthesis of Compound (A-16) is shown in the following. 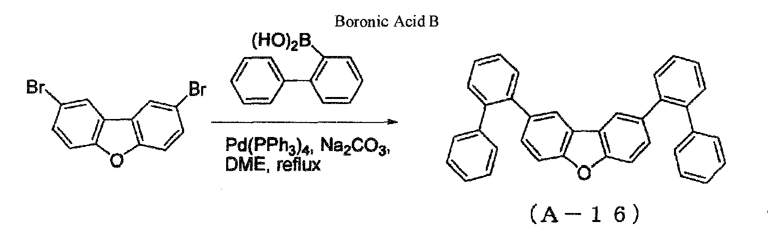 Under the atmosphere of argon gas, 2,8-dibromodibenzofuran in an amount of 1.87 g (5.74 mmol), boronic acid B in an amount of 2.70 g (13.7 mmol) and tetrakis(triphenylphosphine)palladium in an amount of 0.792 g (0.685 mmol) were placed into a three neck flask with a capacity of 300 milliliter, and the air inside the flask was replaced with argon gas. Adding 1,2-dimethoxyethane in an amount of 41.1 milliliter and 2.0 M sodium carbonate aqueous solution in an amount of 20.6 milliliter (41.1 mmol) into the flask, the resultant solution was refluxed by heating for 10 hours under the atmosphere of argon gas. Water in an amount of 100 milliliter and methylene chloride in an amount of 100 milliliter were added to the resultant reaction solution, and an organic layer was separated, followed by drying with the use of anhydride magnesium sulfide. After concentrating the dried mixture under reduced pressure by means of an evaporator, a resultant solid was purified by means of silicagel column chromatography (dissolution solvent: methylene chloride / hexane= 1/2) and as a result, 2.03 g (4.31 mmol; the yield: 75 %) of Compound (A-16) was obtained. The compound was confirmed as the aimed Compound (A-16) from the result in accordance with Field Desorption Mass Spectrum (FD-MS) analysis. The result of the measurement in accordance with FD-MS is shown as the following:
FD-MS: calcd for C₃₆H₂₄O= 472, found: m/ z= 472 (M⁺, 100)

### Synthesis Example 5

The route for synthesis of Compound (B-19) is shown in the following. 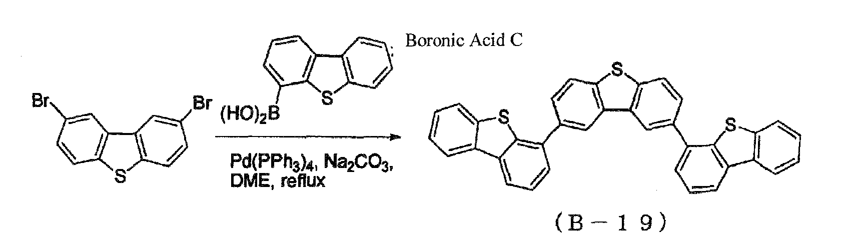 Under the atmosphere of argon gas, 2,8-dibromodibenzothiophene in an amount of 2.13 g (6.29 mmol), boronic acid C in an amount of 3.38 g (15.0 mmol) and tetrakis(triphenylphosphine)palladium in an amount of 0.855 g (0.740 mmol) were placed into a three neck flask with a capacity of 300 milliliter, and the air inside the flask was replaced with argon gas. Adding 1,2-dimethoxyethane in an amount of 44 milliliter and 2.0 M sodium carbonate aqueous solution in an amount of 22 milliliter (44.4 mmol) into the flask, the resultant solution was refluxed by heating for 10 hours under the atmosphere of argon gas. After filtering the reaction solution, a resultant solid was washed with a use of water, methanol and methylene chloride, and as a result, 2.15 g (3.92 mmol; the yield: 63 %) of Compound (B-19) was obtained. The compound was confirmed as the aimed Compound (B-19) from the result in accordance with Field Desorption Mass Spectrum (FD-MS) analysis. The result of the measurement in accordance with FD-MS is shown as the following:
FD-MS: calcd for C₃₆H₂₀S₃= 548, found: m/z= 548 (M⁺, 100)

### Synthesis Example 6

The route for synthesis of Compound (A-19) is shown in the following.

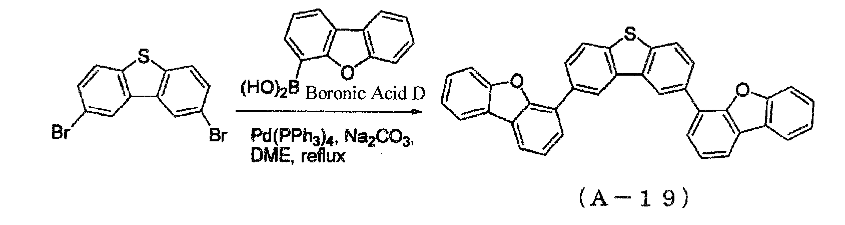

Under the atmosphere of argon gas, 2,8-dibromodibenzothiophene in an amount of 3.52 g (10.3 mmol), boronic acid D in an amount of 5.19 g (24.5 mmol) and tetrakis(triphenylphosphine)palladium in an amount of 1.42 g (1.23 mmol) were placed into a three neck flask with a capacity of 300 milliliter, and the air inside the flask was replaced with argon gas. Adding 1,2-dimethoxyethane in an amount of 73.5 milliliter and 2.0 M sodium carbonate aqueous solution in an amount of 36.8 milliliter (73.5 mmol) into the flask, the resultant solution was refluxed by heating for 10 hours under the atmosphere of argon gas. After filtering the reaction solution, a resultant solid was washed with a use of water, methanol and methylene chloride, and as a result, 3.78 g (7.31 mmol; the yield: 71 %) of Compound (A-19) was obtained. The compound was confirmed as the aimed Compound (A-19) from the result in accordance with Field Desorption Mass Spectrum (FD-MS) analysis. The result of the measurement in accordance with FD-MS is shown as the following:
FD-MS: calcd for C₃₆H₂₀O₂S= 516, found: m/z= 516 (M⁺, 100)

### Synthesis Example 7

The route for synthesis of Compound (A-9) is shown in the following. 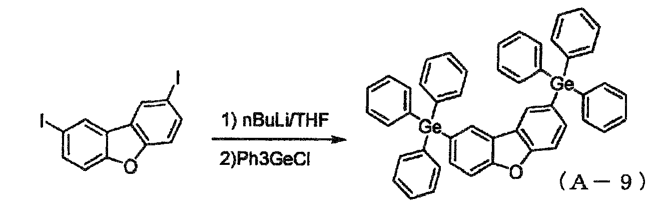 Under the atmosphere of argon gas, 2,8-diiododibenzofuran in an amount of 3.0 g (7.16 mmol) and dehydrated tetrahydrofuran D in an amount of 100 milliliter were placed into a three neck flask with a capacity of 300 milliliter, and the temperature of the resultant solution was cooled down to -70 °C while stirring. Further, 9.9 milliliter (15.8 mmol) of a solution prepared by dissolving normal butyllithium 1.6M into hexane was added and then, spending 30 minutes, the temperature of the resultant solution was elevated up to -10 °C while stirring. Subsequently, the temperature of the solution was cooled down to -70 °C again and afterwards, a solution prepared by dissolving triphenyl germanium chloride in an amount of 4.7 g (16 mmol) into 50 milliliter of dehydrated tetrahydrofuran was dripped down spending 10 minutes, and the temperature of the reacted solution was elevated up to a room temperature spending 1 hour. Further, adding a saturated ammonium chloride aqueous solution, the reaction was completed. Extracting with a use of dichloro-methane, the resultant mixture was concentrated and then, a resultant solid was purified by means of silicagel column chromatography and resultantly, 1.92 g (2.47 mmol; the yield: 35 %) of Compound (A-9) was obtained. The compound was confirmed as the aimed Compound (A-9) from the result in accordance with Field Desorption Mass Spectrum (FD-MS) analysis. The result of the measurement in accordance with FD-MS is shown as the following:
FD-MS: calcd for C₄₈H₃₆Ge₂O= 776, found: m/z= 776, 774

### Synthesis Example 8

The route for synthesis of Compound (B-9) is shown in the following. 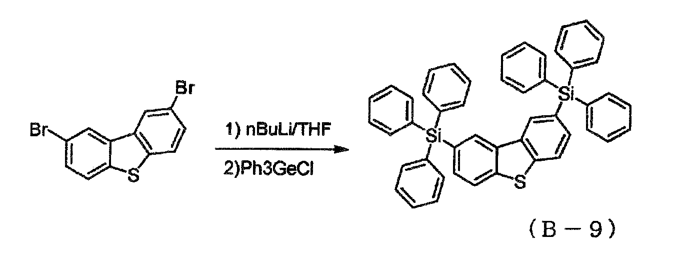 Under the atmosphere of argon gas, 2,8-dibromodibenzothiophene in an amount of 2.0 g (5.85 mmol) and dehydrated tetrahydrofuran in an amount of 80 milliliter were placed into a three neck flask with a capacity of 300 milliliter, and the temperature of the resultant solution was cooled down to -70 °C while stirring. Further, 8.0 milliliter (12.8 mmol) of a solution prepared by dissolving normal butyllithium 1.6M into hexane was added and then, spending 30 minutes, the temperature of the resultant solution was elevated up to -10 °C while stirring. Subsequently, the temperature of the solution was cooled down to -70 °C again and afterwards, a solution prepared by dissolving triphenylsilylchloride in an amount of 3.8 g (13 mmol) into 40 milliliter of dehydrated tetrahydrofuran was dripped down spending 10 minutes, and the temperature of the reacted solution was elevated up to a room temperature spending 1 hour. Further, adding a saturated ammonium chloride aqueous solution, the reaction was completed. Extracting with a use of dichloro-methane, the resultant mixture was concentrated and then, a resultant solid was purified by means of silicagel column chromatography and resultantly, 1.84 g (2.62 mmol; the yield: 45 %) of Compound (B-9) was obtained. The compound was confirmed as the aimed Compound (B-9) from the result in accordance with Field Desorption Mass Spectrum (FD-MS) analysis. The result of the measurement in accordance with FD-MS is shown as the following:
FD-MS: calcd for C₄₈H₃₆SSi₂= 700, found: m/z= 700 (M⁺, 100)

### Synthesis Example 9

The route for synthesis of Compound (B-14) is shown in the following. 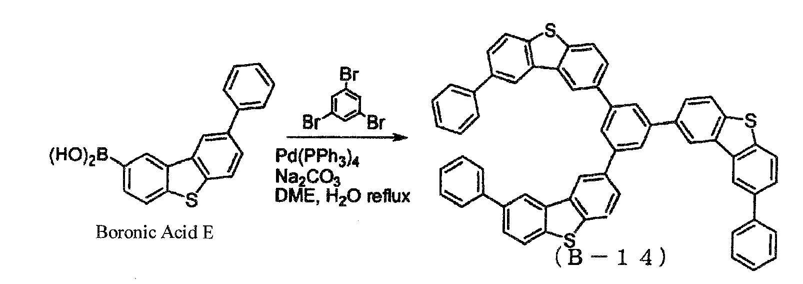 Under the atmosphere of argon gas, boronic acid E in an amount of 4.0 g (13.2 mmol) and tetrakis(triphenylphosphine)palladium in an amount of 760 mg (0.66 mmol) and tribromobenzene in an amount of 940 mg (3.0 mmol) were placed into a three neck flask with a capacity of 300 milliliter, and the air inside the flask was replaced with argon gas. Adding 1,2-dimethoxyethane in an amount of 40 milliliter and 2.0 M sodium carbonate aqueous solution in an amount of 20 milliliter (40 mmol) into the flask, the resultant solution was refluxed by heating for 12 hours under the atmosphere of argon gas. Extracting the reacted solution with a use of dichloro-methane, the resultant mixture was concentrated and then, a resultant solid was purified by means of silicagel column chromatography and resultantly, 1.13 g (1.33 mmol; the yield: 44 %) of Compound (B-14) was obtained. The compound was confirmed as the aimed Compound (B-14) from the result in accordance with Field Desorption Mass Spectrum (FD-MS) analysis. The result of the measurement in accordance with FD-MS is shown as the following:
FD-MS: calcd for C₆₀H₃₆S₃= 852, found: m/z= 853, 852

### Synthesis Example 10

The route for synthesis of Compound (C-1) is shown in the following. 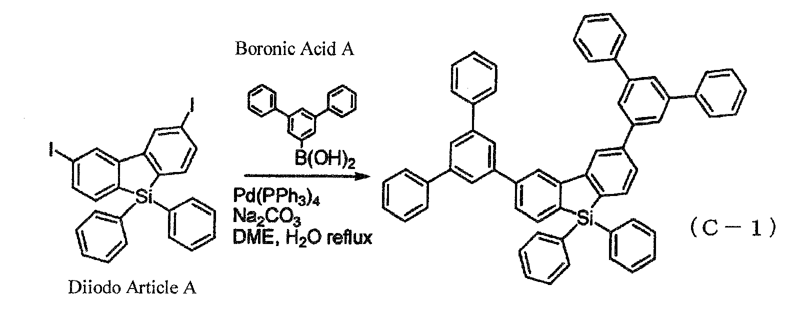 Under the atmosphere of argon gas, diiodo article A in an amount of 3.6 g (6.1 mmol), boronic acid A in an amount of 3.67 g (13.4 mmol) and tetrakis(triphenylphosphine)palladium in an amount of 770 mg (0.67 mmol) were placed into a three neck flask with a capacity of 300 milliliter, and the air inside the flask was replaced with argon gas. Adding 1,2-dimethoxyethane in an amount of 50 milliliter and 2.0 M sodium carbonate aqueous solution in an amount of 20 milliliter (40 mmol) into the flask, the resultant solution was refluxed by heating for 12 hours under the atmosphere of argon gas. Water in an amount of 100 milliliter and methylene chloride in an amount of 100 milliliter were added to the resultant reaction solution, and after extraction, the resultant substance was concentrated under reduced pressure by means of an evaporator. The resultant solid was purified by means of silicagel column chromatography and resultantly, 2.12 g (2.68 mmol; the yield: 44 %) of Compound (C-1) was obtained. The compound was confirmed as the aimed Compound (C-1) from the result in accordance with Field Desorption Mass Spectrum (FD-MS) analysis. The result of the measurement in accordance with FD-MS is shown as the following:
FD-MS: calcd for C₆₀H₄₂Si= 790, found: m/z= 790 (M⁺, 100%)

### Synthesis Example 11

The route for synthesis of Compound (D-6) is shown in the following.

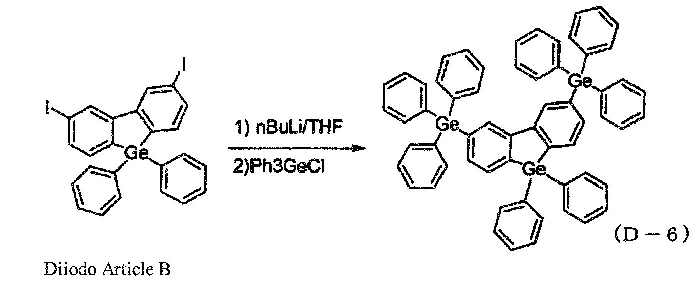

Under the atmosphere of argon gas, diiodo article B in an amount of 3.5 g (5.6 mmol) and dehydrated tetrahydrofuran in an amount of 80 milliliter were placed into a three neck flask with a capacity of 300 milliliter, and the temperature of the resultant solution was cooled down to -70 °C while stirring. Further, 7.8 milliliter (12.3 mmol) of a solution prepared by dissolving normal butyllithium 1.6M into hexane was added and then, spending 30 minutes, the temperature of the resultant solution was elevated up to -10 °C while stirring. Subsequently, the temperature of the solution was cooled down to -70 °C again and afterwards, a solution prepared by dissolving triphenylgermaniumchloride in an amount of 4.4 g (13 mmol) into 50 milliliter of dehydrated tetrahydrofuran was dripped down spending 10 minutes, and the temperature of the reacted solution was elevated up to a room temperature spending 1 hour. Further, adding a saturated ammonium chloride aqueous solution, the reaction was completed. Extracting with a use of dichloro-methane, the resultant mixture was concentrated and then, a resultant solid was purified by means of silicagel column chromatography and resultantly, 2.63 g (2.66 mmol; the yield: 48 %) of Compound (D-6) was obtained. The compound was confirmed as the aimed Compound (D-6) from the result in accordance with Field Desorption Mass Spectrum (FD-MS) analysis. The result of the measurement in accordance with FD-MS is shown as the following:
FD-MS: calcd for C₆₀H₄₆Ge₃= 988, found: m/z= 988, 986, 984

Additionally, an apparatus used for the measurement and measurement conditions of Field Desorption Mass Spectrometry (FD-MS) analysis in Synthesis Examples 1 to 11 will be described bellow.

### <FD-MS measurement>

Apparatus: HX110 (produced by JEOL Ltd.)
Conditions: Accelerating voltage 8 kV
   Scanning range m/z= 50 to 1500
Material of Emitter: Carbon
Emitter current: 0 mA → 2 mA / minute → 40 mA
   (Maintaining for 10 minutes)

The minimum exciting triplet energy level: T₁ of the compounds were measured in accordance with the following methods.
Employing EPA (diethyl ether : isopentane : isopropanol= 5 : 5 : 2 in volume ratio) as a solvent, with a concentration of 10 µmol/liter, at a temperature of 77 K, a triplet energy level was measured using quartz cell by means of SPEX FLUOROLOGII. A tangent was drawn to the increasing line at the short wavelength side of the phosphorescence spectrum, and the wavelength (the end of light emission) at the intersection of the tangent and the abscissa was obtained. The obtained wavelength was converted into the energy.
Measurement results of triplet energy level about the synthesized compounds are shown respectively in Table 1.

**Table 1 Triplet energy level**

| Compound | Triplet energy level (eV) |
|---|---|
| A-1 | 2.86 |
| B-1 | 2.86 |
| A-16 | 2.84 |
| B-16 | 2.84 |
| A-19 | 2.83 |
| B-19 | 2.82 |
| A-9 | 2.98 |
| B-9 | 3.03 |
| B-14 | 2.84 |
| C-1 | 2.61 |
| D-6 | 2.60 |

Fabrication examples of the organic EL device will be shown below.
All the compounds obtained by the synthesis processes in the foregoing
Synthesis Examples are used after purifying by sublimation under a reduced pressure of 10⁻¹ to 10⁻⁴ Pa.

### Example 1 (Fabrication of an organic EL device)

A glass substrate (manufactured by GEOMATEC Company) of 25 mmx75 mm×1.1 mm thickness having an ITO transparent electrode was cleaned by application of ultrasonic wave in isopropyl alcohol for 5 minutes and then by exposure to ozone generated by ultraviolet light for 30 minutes. The glass substrate having the transparent electrode which had been cleaned was attached to a substrate holder of a vacuum vapor deposition apparatus. On the surface of the cleaned substrate at the side having the transparent electrode, a film of HTM (refer to a chemical formula below) having a thickness of 100 nm was formed so that the formed film covered the transparent electrode. The formed film of HTM worked as the hole injecting and transporting layer. Then, subsequent to the film formation of the hole injecting and transporting layer, a film of the host Compound (A-1) and Complex (K-1) having a thickness of 30 nm was formed by jointly vapor depositing on the formed film of HTM. The concentration of Complex (K-1) was 7 % by weight. The film of the host Compound (A-1) worked as the light emitting layer. Then, subsequent to the film formation of the light emitting layer, a film of a material ETM1 below having a thickness of 25 nm was formed and further on the film of ETM1, a film of a material ETM2 below having a thickness of 5 nm, was formed by laminating. The film of ETM1 and the film of ETM2 each independently worked as an electron transporting layer and an electron injecting layer respectively. Subsequently, lithium fluoride was deposited with a film-forming rate of 1 Å/minute up to 0.1 nm in thickness resultantly making an electron injecting electrode (cathode). On the lithium fluoride film, aluminum was vapor deposited to form a metal cathode having a thickness of 150 nm and an organic EL device was fabricated.

### (Luminescent property evaluation about the organic EL device)

The organic EL device fabricated above was lit by direct current drive (current density J= 1mA /cm²), and Luminance (L) was measured, followed by calculating Current Efficiency (L/J) and the resultants are shown in Table 2.

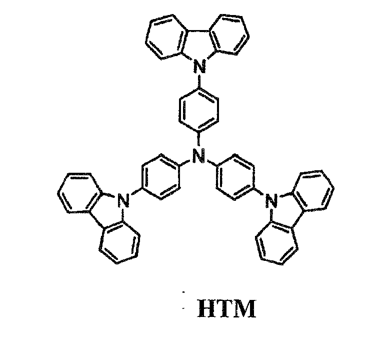

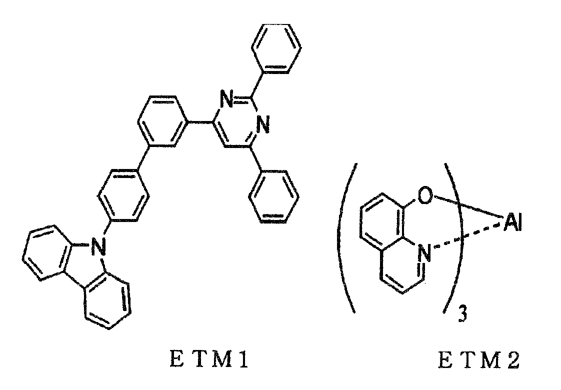

### Examples 2 to 9

Organic EL devices were fabricated in the same manners as Example 1 except that compounds described in Table 2 were employed instead of the host Compound (A-1). Current efficiencies were measured respectively about the resultant organic EL devices in the same manners as Example 1, and the measured results are shown in Table 2.

### Comparative Examples 1 to 3

Organic EL devices were fabricated in the same manners as Example 1 except that Reference Compounds 1 to 3 below each described in Japanese Unexamined Patent Application Laid-Open Nos. 5-109485, 2004-002351 and 2002-308837 were employed instead of the host Compound (A-1). Current efficiencies were measured respectively about the resultant organic EL devices in the same manners as Example 1, and the measured results are shown in Table 2.

### Examples 10 and 11

Organic EL devices were fabricated in similar manners as Example 1 except that Complex (K-17) and compounds described in a column of Host Compound on Table 3 were employed instead of Complex (K-1) and the host Compound (A-1) respectively. Current efficiencies were measured respectively about the resultant organic EL devices in the same manners as Example 1, and the measured results are shown in Table 3.

### Comparative Examples 5 to 7

Organic EL devices were fabricated in the same manners as Example 10 except that Reference Compounds 1 to 3 below each described in Japanese Unexamined Patent Application Laid-Open Nos. 5-109485, 2004-002351 and 2002-308837 were employed instead of the host Compound (A-1). Current efficiencies were measured respectively about the resultant organic EL devices in the same manners as Example 1, and the measured results are shown in Table 3.

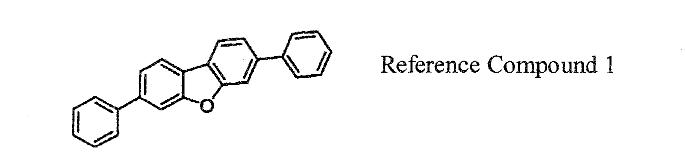

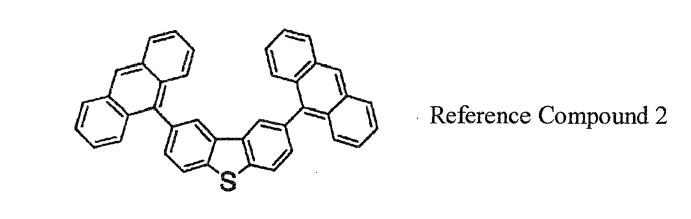

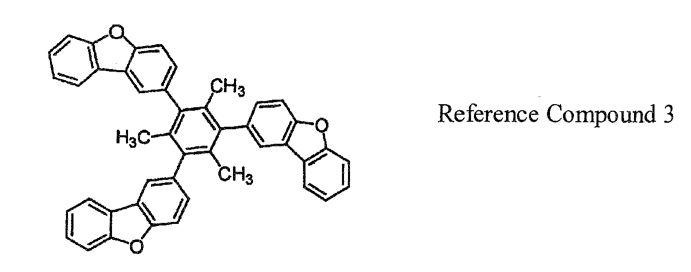

In Tables 2 and 3 below, λ max means the maximum wavelength of light emission.

**Table 2**

| | Host Compound | J | L | L/J | λmax |
|---|---|---|---|---|---|
| | Number | (mA/cm²) | (cd/m²) | (cd/A) | (nm) |
| Example 1 | A-1 | 1.0 | 466 | 47 | 487 |
| Example 2 | B-1 | 1.0 | 418 | 42 | 486 |
| Example 3 | A-16 | 1.0 | 321 | 32 | 487 |
| Example 4 | B-16 | 1.0 | 350 | 35 | 486 |
| Example 5 | A-19 | 1.0 | 487 | 49 | 487 |
| Example 6 | B-19 | 1.0 | 491 | 49 | 487 |
| Example 7 | A-9 | 1.0 | 320 | 32 | 485 |
| Example 8 | B-9 | 1.0 | 304 | 30 | 485 |
| Example 9 | B-14 | 1.0 | 438 | 44 | 486 |
| Comparative Example 1 | Reference Compound 1 | 1.0 | 102 | 10 | 487 |
| Comparative Example 2 | Reference Compound 2 | 1.0 | - | No light emission | - |
| Comparative Example 3 | Reference Compound 3 | 1.0 | 228 | 23 | 487 |

**Table 3**

| | Host Compound | J | L | L/J | λmax |
|---|---|---|---|---|---|
| | Number | (mA/cm²) | (cd/m²) | (cd/A) | (nm) |
| Example 10 | A-9 | 1.0 | 98 | 10 | 440 |
| Example 11 | B-9 | 1.0 | 128 | 13 | 440 |
| Comparative Example 4 | Reference Compound 1 | 1.0 | - | No light emission | - |
| Comparative Example 5 | Reference Compound 2 | 1.0 | - | No light emission | - |
| Comparative Example 6 | Reference Compound 3 | 1.0 | 34 | 3 | 441 |

Surveying about Tables 2 and 3 verifies that the organic EL devices employing the compounds of the present invention for a light emitting layer exhibit enhanced current efficiencies. The results also verify that the compounds in the present invention are effective for a purpose of using in the organic EL devices.

### INDUSTRIAL APPLICABILITY

As explained above, employing the compound represented by any one of general formulae (1) to (7) of the present invention as the material for an organic electroluminescence device provides the organic electroluminescence device with an enhanced current efficiency of light emission, without any pixel defects, with superiority in heat resistance and with prolonged lifetime. Therefore, the organic EL device of the present invention is very useful for applications such as light sources of various electronic instruments.

## Claims

1. A material for an organic electroluminescence device which comprises a compound represented by a following general formula (1): 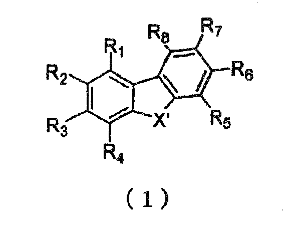 wherein R₁ to R₈ each independently represents a hydrogen atom, a halogen atom, an alkyl group having 1 to 40 carbon atoms and further may have a substituent, a heterocyclic group except pyridine ring while the heterocyclic group has 3 to 20 carbon atoms and further may have a substituent, an alkoxy group having 1 to 40 carbon atoms and further may have a substituent, a non-condensed aryl group having 6 to 40 carbon atoms and further may have a substituent, a condensed aryl group having 6 to 12 carbon atoms and further may have a substituent, a mixed aryl group of a condensed aryl group and a non-condensed aryl group while the mixed aryl group has 12 to 40 carbon atoms and further may have a substituent, an aryloxy group having 6 to 20 carbon atoms and further may have a substituent, an aralkyl group having 7 to 20 carbon atoms and further may have a substituent, an alkenyl group having 2 to 40 carbon atoms and further may have a substituent, an alkylamino group having 1 to 40 carbon atoms and further may have a substituent, an aralkylamino group having 7 to 60 carbon atoms and further may have a substituent, an alkylsilyl group having 3 to 20 carbon atoms and further may have a substituent, an arylsilyl group having 8 to 40 carbon atoms and further may have a substituent, an aralkylsilyl group having 8 to 40 carbon atoms and further may have a substituent, an alkylgermanium group having 3 to 20 carbon atoms and further may have a substituent, an arylgermanium group having 8 to 40 carbon atoms and further may have a substituent, an aralkylgermanium group having 8 to 40 carbon atoms and further may have a substituent, a keto aryl group having 7 to 40 carbon atoms and further may have a substituent, an alkylhalide group having 1 to 40 carbon atoms and further may have a substituent, or a cyano group;
X' represents a sulfur atom, an oxygen atom or a substituted germanium group expressed by GeR_{c}R_{d}, while R_{c} and R_{d} each independently represents an alkyl group having 1 to 40 carbon atoms or an aryl group having 6 to 20 carbon atoms; however, at least one of R₂ or R₇ independently represents a non-fused aromatic ring having 6 to 40 carbon atoms and further may have a substituent, a naphthyl group which may have a substituent, an alkylsilyl group having 3 to 20 carbon atoms and further may have a substituent, an arylsilyl group having 8 to 40 carbon atoms and further may have a substituent, an aralkylsilyl group having 8 to 40 carbon atoms and further may have a substituent, an alkylgermanium group having 3 to 20 carbon atoms and further may have a substituent, an arylgermanium group having 8 to 40 carbon atoms and further may have a substituent or an aralkylgermanium group having 8 to 40 carbon atoms and further may have a substituent; and
each of R₂ and R₇ is not an amino group.

2. A material for an organic electroluminescence device which comprises a compound represented by a following general formula (2) or a following general formula (3): 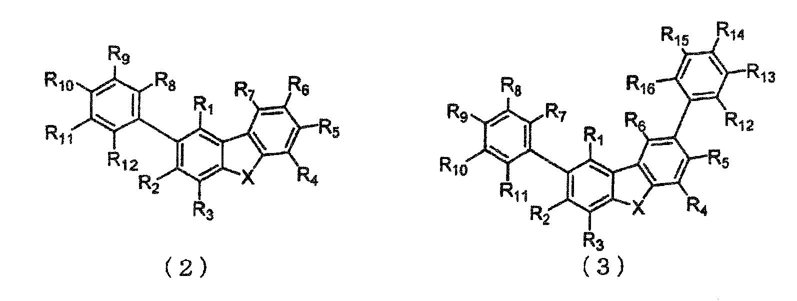 wherein R₁ to R₁₆ each independently represents a hydrogen atom, a halogen atom, an alkyl group having 1 to 40 carbon atoms and further may have a substituent, a heterocyclic group having 3 to 20 carbon atoms and further may have a substituent, an alkoxy group having 1 to 40 carbon atoms and further may have a substituent, a non-condensed aryl group having 6 to 40 carbon atoms and further may have a substituent, a condensed aryl group having 6 to 12 carbon atoms and further may have a substituent, a mixed aryl group of a condensed aryl group and a non-condensed aryl group while the mixed aryl group has 12 to 40 carbon atoms and further may have a substituent, an aryloxy group having 6 to 20 carbon atoms and further may have a substituent, an aralkyl group having 7 to 20 carbon atoms and further may have a substituent, an alkenyl group having 2 to 40 carbon atoms and further may have a substituent, an alkylamino group having 1 to 40 carbon atoms and further may have a substituent, an aralkylamino group having 7 to 60 carbon atoms and further may have a substituent, an alkylsilyl group having 3 to 20 carbon atoms and further may have a substituent, an arylsilyl group having 8 to 40 carbon atoms and further may have a substituent, an aralkylsilyl group having 8 to 40 carbon atoms and further may have a substituent, an alkylgermanium group having 3 to 20 carbon atoms and further may have a substituent, an arylgermanium group having 8 to 40 carbon atoms and further may have a substituent, an aralkylgermanium group having 8 to 40 carbon atoms and further may have a substituent, a keto aryl group having 7 to 40 carbon atoms and further may have a substituent, an alkylhalide group having 1 to 40 carbon atoms and further may have a substituent, or a cyano group;
X represents a sulfur atom, an oxygen atom, a substituted silicon atom expressed by SiRₐR_{b} or a substituted germanium group expressed by GeR_{c}R_{d}, while Rₐ, R_{b}, R_{c} and R_{d} each independently represents an alkyl group having 1 to 40 carbon atoms or an aryl group having 6 to 20 carbon atoms;
however, each of R₁₀ in the general formula (2), R₉ and R₁₄ in the general formula (3) is not an amino group; and
at least one of R₈ or R₁₂ in the general formula (2) is a hydrogen atom.

3. The material for an organic electroluminescence device according to Claim 2, wherein said material is represented by a following formula (4) or a following general formula (5): 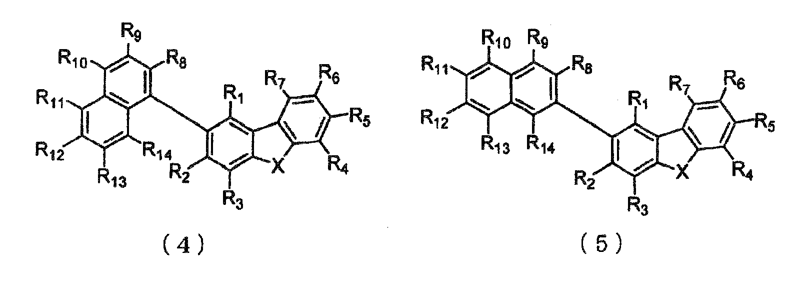 wherein R₁ to R₁₄ each independently represents a hydrogen atom, a halogen atom, an alkyl group having 1 to 40 carbon atoms and further may have a substituent, a heterocyclic group having 3 to 20 carbon atoms and further may have a substituent, an alkoxy group having 1 to 40 carbon atoms and further may have a substituent, a non-condensed aryl group having 6 to 40 carbon atoms and further may have a substituent, a condensed aryl group having 6 to 12 carbon atoms and further may have a substituent, a mixed aryl group of a condensed aryl group and a non-condensed aryl group while the mixed aryl group has 12 to 40 carbon atoms and further may have a substituent, an aryloxy group having 6 to 20 carbon atoms and further may have a substituent, an aralkyl group having 7 to 20 carbon atoms and further may have a substituent, an alkenyl group having 2 to 40 carbon atoms and further may have a substituent, an alkylamino group having 1 to 40 carbon atoms and further may have a substituent, an aralkylamino group having 7 to 60 carbon atoms and further may have a substituent, an alkylsilyl group having 3 to 20 carbon atoms and further may have a substituent, an arylsilyl group having 8 to 40 carbon atoms and further may have a substituent, an aralkylsilyl group having 8 to 40 carbon atoms and further may have a substituent, an alkylgermanium group having 3 to 20 carbon atoms and further may have a substituent, an arylgermanium group having 8 to 40 carbon atoms and further may have a substituent, an aralkylgermanium group having 8 to 40 carbon atoms and further may have a substituent, a keto aryl group having 7 to 40 carbon atoms and further may have a substituent, an alkylhalide group having 1 to 40 carbon atoms and further may have a substituent, or a cyano group; and
X represents a sulfur atom, an oxygen atom, a substituted silicon atom expressed by SiRₐR_{b} or a substituted germanium group expressed by GeR_{c}R_{d}, while Rₐ, R_{b}, R_{c} and R_{d} each independently represents an alkyl group having 1 to 40 carbon atoms or an aryl group having 6 to 20 carbon atoms.

4. A material for an organic electroluminescence device which comprises a compound represented by a following general formula (6) or a following general formula (7): 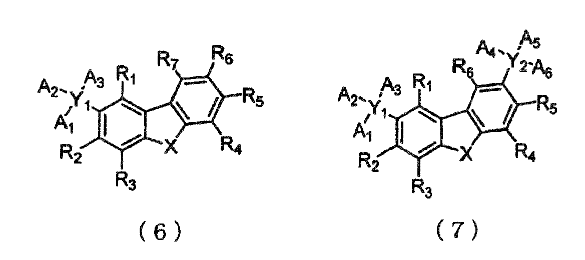 wherein R₁ to R₇ each independently represents a hydrogen atom, a halogen atom, an alkyl group having 1 to 40 carbon atoms and further may have a substituent, a heterocyclic group having 3 to 20 carbon atoms and further may have a substituent, an alkoxy group having 1 to 40 carbon atoms and further may have a substituent, a non-condensed aryl group having 6 to 40 carbon atoms and further may have a substituent, a condensed aryl group having 6 to 12 carbon atoms and further may have a substituent, a mixed aryl group of a condensed aryl group and a non-condensed aryl group while the mixed aryl group has 12 to 40 carbon atoms and further may have a substituent, an aryloxy group having 6 to 20 carbon atoms and further may have a substituent, an aralkyl group having 7 to 20 carbon atoms and further may have a substituent, an alkenyl group having 2 to 40 carbon atoms and further may have a substituent, an alkylamino group having 1 to 40 carbon atoms and further may have a substituent, an aralkylamino group having 7 to 60 carbon atoms and further may have a substituent, an alkylsilyl group having 3 to 20 carbon atoms and further may have a substituent, an arylsilyl group having 8 to 40 carbon atoms and further may have a substituent, an aralkylsilyl group having 8 to 40 carbon atoms and further may have a substituent, an alkylgermanium group having 3 to 20 carbon atoms and further may have a substituent, an arylgermanium group having 8 to 40 carbon atoms and further may have a substituent, an aralkylgermanium group having 8 to 40 carbon atoms and further may have a substituent, a keto aryl group having 7 to 40 carbon atoms and further may have a substituent, an alkylhalide group having 1 to 40 carbon atoms and further may have a substituent, or a cyano group;
X represents a sulfur atom, an oxygen atom, a substituted silicon atom expressed by SiRₐR_{b} or a substituted germanium group expressed by GeR_{c}R_{d}, while Rₐ, R_{b}, R_{c} and R_{d} each independently represents an alkyl group having 1 to 40 carbon atoms or an aryl group having 6 to 20 carbon atoms;
Y₁ and Y₂ each independently represents a silicon atom or a germanium atom; and
A₁ to A₆ each independently represents an alkyl group having 1 to 40 carbon atoms and further may have a substituent, an aryl group having 6 to 40 carbon atoms and further may have a substituent or an aralkyl group having 7 to 20 carbon atoms and further may have a substituent.

5. The material for an organic electroluminescence device according to Claim 1, wherein at least one selected from R₁ to R₈ in the general formula (1) is a mono-valent group derived from dibenzofuran, dibenzothiophene, carbazole, silafluorene, germafluorene or fluorene.

6. The material for an organic electroluminescence device according to Claim 1, wherein at least one of R₁ and R₇ in the general formula (1) is a mono-valent group derived from dibenzofuran, dibenzothiophene, carbazole, silafluorene, germafluorene or fluorene.

7. An organic electroluminescence device comprising an anode, a cathode and at least one organic thin film layer including a light emitting layer sandwiched between the anode and the cathode, wherein at least one of the organic thin film layer comprises the material for an organic electroluminescence device according to any one of Claims 1 to 6.

8. The organic electroluminescence device according to Claim 7, wherein said light emitting layer comprises a host material and a phosphorescent material and wherein the host material comprises the material for an organic electroluminescence device according to any one of Claims 1 to 6.

9. The organic electroluminescence device according to Claim 7, wherein said light emitting layer comprises a host material and a phosphorescent material and wherein the phosphorescent material comprises an iridium metal, an osmium metal or a platinum metal.

10. The organic electroluminescence device according to Claim 7*,* wherein said light emitting layer comprises a host material and a phosphorescent material and wherein the phosphorescent material is a light emitting material having a metal-carbene carbon bond.

11. The organic electroluminescence device according to Claim 7, wherein said material for an organic electroluminescence device is a host material in the light emitting layer of the organic electroluminescence device.

12. The organic electroluminescence device according to Claim 7, which comprises a hole transporting layer and wherein said material for an organic electroluminescence device is a material in the hole transporting layer.

13. The organic electroluminescence device according to Claim 7, which comprises a hole transporting layer or a hole barrier layer and wherein said material for an organic electroluminescence device is a material in the hole transporting layer or in the hole barrier layer.

14. The organic electroluminescence device according to Claim 7, wherein a reductive dopant is added in an interfacial region between said cathode and said organic thin film layer.

15. The organic electroluminescence device according to Claim 7, which further comprises an electron injecting layer between said light emitting layer and said cathode and wherein the electron injecting layer essentially comprises a nitrogen atom-containing ring derivative.

16. The organic electroluminescence device according to Claim 7, wherein said light emitting layer comprises metal complex which enables to emit bluish light with a peak luminance of light emission at a wavelength of 500 nm or shorter.
